# FASCICULE DE BREVET EUROPEEN

(11) **EP 0 981 617 B1**
(45) Date de publication et mention de la délivrance du brevet: **23.07.2008**
(21) Numéro de dépôt: 98924373.8
(22) Date de dépôt: 04.05.1998
(51) Int. Cl.: C12N 15/12, C12N 15/82, C07K 14/79, A01H 5/00

(54) **LACTOFERRINES RECOMBINANTES, LEURS PROCEDES DE PRODUCTION PAR LES PLANTES AINSI QUE LEURS UTILISATIONS**
REKOMBINANTE LACTOFERRIN, VERFAHREN ZU IHRER HERSTELLUNG MITTELS PFLANZEN UND IHRE VERWENDUNG
RECOMBINANT LACTOFERRIN, METHODS OF PRODUCTION FROM PLANTS AND USES

(30) Priorité: 02.05.1997 FR 9705699
(43) Date de publication de la demande: 01.03.2000
(73) Titulaire: MERISTEM THERAPEUTICS S.A., 63100 Clermont-Ferrand (FR)
(72) Inventeur: LEGRAND, Dominique, F-59650 Villeneuve d'Ascq (FR); SALMON, Valérie, F-59780 Willems (FR); SPIK, Geneviève, F-59700 Marcq en Baroeul (FR); GRUBER, Véronique Résidence Sainte Madeleine, F-63400 Chamalières (FR); BOURNAT, Philippe, F-63000 Clermont-Ferrand (FR); MEROT, Bertrand, F-63530 Volvic (FR)
(74) Mandataire: Myon, Gérard Jean-Pierre
(86) Numéro de dépôt international: PCT/FR1998/000895
(87) Numéro de publication internationale: WO 1998/050543

(56) Documents cités:
- WO-A-95/30339
- WO-A-96/37094
- WO-A-98/06860
- FR-A- 2 736 930
- ZHANG, ZHANYUAN [PH.D.] ET AL: "DEVELOPMENT OF TRANSGENIC PLANTS WITH NON-PLANT ANTIBACTERIAL PROTEIN GENES FOR RESISTANCE TO BACTERIAL PATHOGENS ( LACTOFERRIN, NICOTIANA TABACUM, AGROBACTERIUM TUMEFACIENS)" PHD DISSERTATION, UNIVERSITY OF NEBRASKA, juin 1996, XP002078826
- ZHANG, Z., ET AL.: "Gene transfer for enhancing plant disease-resistance to bacterial pathogens" HORTSCIENCE, vol. 30, no. 4, 1995, page 788 XP002078827
- MITRA A ET AL: "Expression of of a human lactoferrin cDNA in tobacco cells produces antibacterial protein(s)." PLANT PHYSIOLOGY (ROCKVILLE) 106 (3). 1994. 977-981. ISSN: 0032-0889, XP002048441
- WARD, P.P., ET AL.: "A system for production of commercial quantities of human lactoferrin: a broad spectrum natural antibiotic" BIOTECHNOLOGY, vol. 13, mai 1995, pages 498-503, XP002048442
- DIERYCK W ET AL: "EXPRESSION D'HEMOGLOBINE HUMAINE RECOMBINANTE DANS LES PLANTES RECOMBINANT PROTEIN EXPRESSION IN PLANTS" TRANSFUSION CLINIQUE ET BIOLOGIQUE, vol. 2, no. 6, 1995, pages 441-447, XP000614371

## Description

La présente invention concerne des lactoferrines recombinantes (rLf), leurs procédés de production par les plantes ainsi que leurs utilisations.

L'utilisation de certaines protéines issues de mammifères est remise en question en raison des risques éventuels de contamination par des agents infectieux non conventionnels, notamment de type prions. Les conséquences sur la commercialisation et les dispositions réglementaires sont lourdes. La mise au point de la production de protéines exemptes de toute contamination animale est donc un nouvel enjeu mais elle rend compte de difficultés nouvelles, qui sont fonction de la protéine et du matériel végétal choisis.
On connaît dans l'art antérieur la publication de Mitra et al (1994) qui décrit la transformation de cellules de tabac avec la séquence d'ADN codant pour la lactoferrine humaine. Cependant, cette publication est limitée aux cellules végétales et ne permet pas d'obtenir des plantes régénérées à partir de ces cellules. En outre, la protéine exprimée n'est pas purifiée et semble de toute façon imparfaite puisque seul un fragment (48 kDa) est détecté au lieu de la protéine entière.

On connaît également dans l'art antérieur la demande WO 9637094 qui décrit l'obtention de plantes résistantes aux virus en les transformant avec un gène codant pour la lactoferrine. Cependant, dans ce document également, la protéine n'est pas purifiée et le seul indice de sa présence est un test Western blot.
De par la complexité de la lactoferrine et les particularités propres au matériel végétal utilisé, les étapes d'extraction et de purification représentent également un obstacle majeur à l'obtention de lactoferrine à partir de plantes. Les difficultés soulevées par ces deux étapes peuvent constituer une problématique nouvelle pour chaque protéine que l'on désire obtenir à partir d'une plante.

La lactoferrine est une glycoprotéine de la famille des transferrines. Après sa découverte dans le lait de femme, la lactoferrine fut mise en évidence dans les laits de nombreuses autres espèces animales comme la vache, la chèvre, la truie, la souris et le cobaye, mais à des concentrations fort variables. Dans le lait de femme, la concentration de lactoferrine est de 1 à 2 g/l ; celle-ci est particulièrement élevée dans le colostrum puis diminue au cours de la lactation. Dans le lait, la lactoferrine est présente principalement sous forme apo, c'est-à-dire non saturée en fer. La lactoferrine a, d'autre part, été découverte dans de nombreuses autres sécrétions telles que la salive, la bile, le suc pancréatique et les sécrétions de l'intestin grêle. On la retrouve dans la plupart des mucus comme les sécrétions bronchiques, les sécrétions vaginales, nasales et intestinales.

La lactoferrine est présente également dans les leucocytes neutrophiles polymorphonucléaires où elle est localisée dans les granules secondaires des cellules ne possédant pas de myelopéroxydase. La lactoferrine leucocytaire est synthétisée au cours de la granulopoïèse du stade promyélocytaire au stade métamyélocytaire. Lors de la dégranulation des neutrophiles, la lactoferrine se retrouve libérée dans le plasma à une concentration relativement faible (0,4 à 2 mg/1), comparativement au taux de transferrine présente dans le sang (2 à 3 g/1).

Dès 1984, Metz-Boutigue *et al*. ont déterminé la séquence peptidique de la lactoferrine humaine (hLf). Cette séquence de 692 acides aminés a été confirmée par le clonage de l'ADNc de la lactoferrine de glande mammaire humaine (Powell et Ogden, 1990, Rey *et al.,* 1990). La lactoferrine et la sérotransferrine possèdent une structure primaire et une conformation spatiale très proches. Ainsi, leur chaîne polypeptidique est formée de 2 lobes (lobe N terminal et lobe C terminal) reliés par un petit peptide en hélice alpha. Les homologies de séquence entre les moitiés N et C terminales de la lactoferrine humaine atteignent 37%. Par hydrolyse trypsique de la lactoferrine humaine, Legrand et *al.* (1984) ont obtenu le fragment N trypsique (N-t) de 30 kDa (résidus d'acides aminés 4 à 283), et le fragment C trypsique (C-t) de 50 kDa (résidus d'acides aminés 284 à 692). En proportion équimolaire, ces fragments sont capables de se réassocier en un complexe non covalent N-t / C-t qui possède des propriétés électrophorétiques et spectroscopiques voisines de celle de la lactoferrine humaine (Legrand et *al.,* 1986).

La lactoferrine peut fixer de manière réversible 2 ions ferriques en développant une coloration rose saumon dont le maximum d'absorption est centré à 465 nm. La fixation de chaque ion ferrique nécessite celle d'un ion carbonate. A pH 6,4, la constante d'association pour le complexe [Fe3+]2-Lf est de l'ordre de 10²⁴ M⁻¹ mais diminue avec le pH. Des études par diffraction des rayons X de la lactoferrine humaine à 3,2 Å, à 2,8 Å et à 2,2 Å ont montré que chaque ion ferrique est coordinné à 2 résidus de tyrosine, une histidine, un acide aspartique et un ion carbonate. Ces acides aminés sont les mêmes dans chacun des 2 lobes. Les 2 sites de fixation du fer de la lactoferrine possèdent une forte affinité pour ce métal mais le libèrent à des pH différents. Ainsi, le lobe N-t libère son fer à pH 5,8 (lobe acido labile), alors que le lobe C-t (lobe acido-stable) relargue son fer à pH 4. D'autres ions sont susceptibles de se fixer à la protéine, en particulier le gallium. Des chercheurs se sont intéressés à l'utilisation du complexe ⁶⁷Ga-Lf comme traceur dans les diagnostiques anti-cancer et ont montré qu'après injection de ⁶⁷Ga, le complexe se retrouve préférentiellement dans les tissus mammaires, dans les sécrétions physiologiques et pathologiques, ainsi que dans les lymphomes de Burkitt et Hodgkin.

En ce qui concerne la glycosylation, la lactoferrine isolée du lait de femme porte 3 sites de glycolysation qui sont l'Asn¹³⁸ l'Asn⁴⁷⁹et l'Asn⁶²⁴, le premier étant situé dans le lobe N-t et les deux autres dans le C-t. La glycosylation s'effectue préférentiellement sur 2 sites (Asn¹³⁸ et Asn⁴⁷⁹) dans 85 % des molécules alors que la glycosylation d'un site (Asn⁴⁷⁹) et des trois sites simultanément se produit dans 5 et 9 % des cas respectivement. Les glycannes de la lactoferrine sont de type N-acétyllactosaminique mono ou disialylés et fucosylés (Spik *et al.,1982).* Les résidus de fucose sont branchés en α (1,6) sur la N-acétylglucosamine du point d'attache ou en α (1,3) sur la N-acétylglucosamine 5' de l'antenne. La lactoferrine leucocytaire diffère de la précédente par l'absence totale de fucose.

Alors que la sérotransferrine est incontestablement le principal transporteur de fer de l'ensemble des cellules de l'organisme, les rôles de la lactoferrine paraissent essentiellement liés à la défense de l'organisme et aux mécanismes inflammatoires, en agissant soit directement sur les micro-organismes pathogènes, soit indirectement sur les cellules immunitaires effectrices.

La lactoferrine est un agent antimicrobien dont l'action met en jeu plusieurs mécanismes. Le premier est l'effet bactériostatique de la lactoferrine par ferriprivation (Spik et *al.,* 1978). En séquestrant le fer du milieu environnant, la lactoferrine inhibe la division des bactéries, le fer étant un élément indispensable à la biosynthèse de l'ADN. De plus, un mécanisme d'action plus complexe faisant intervenir des anticorps a été démontré. En effet, l'activité bactériostatique de la lactoferrine augmente en présence d'IgA et d'IgG spécifiques des bactéries pathogènes. D'autre part, le lysozyme peut associer son activité lytique pour les parois des bactéries Gram + à l'action de la lactoferrine. Ainsi, dans le lait, la lactoferrine, le lyzozyme et les anticorps peuvent agir en synergie lors d'une attaque microbienne.

Le second effet antibactérien de la lactoferrine est lié à son pouvoir bactéricide. La lactoferrine se fixerait à la paroi des bactéries Gram- ce qui la déstabiliserait et provoquerait une libération des lipopolysaccharides (LPS). Les parois deviendraient ainsi plus fragiles et plus sensibles à l'action d'antibiotiques hydrophobes. Ces hypothèses ont été confirmées par l'utilisation de la microscopie électronique qui montre l'effet déstabilisateur de la lactoferrine sur les bactéries Gram-, dont *E*. *coli*. Une hypothèse a été émise selon laquelle la fixation de la lactoferrine s'effectuerait sur le lipide A des LPS, et qu'elle serait suivie par l'extraction de ces LPS de la membrane externe des bactéries, en les endommageant irrémédiablement. La région bactéricide de la lactoferrine humaine (lactoferricine A) et bovine (lactoferricine B) a été identifiée récemment: elles sont toutes deux localisées dans une boucle du lobe N-terminal, comprenant 18 acides aminés, boucle formée par un pont disulfure entre les résidus de Cys 20 et 37 pour la lactoferrine humaine et 19 à 36 pour la lactoferrine bovine. En outre, l'importance de la boucle formée par les residus 28-34 de la lactoferrine humaine dans sa fixation aux LPS a été montrée.

Ainsi, par ses activités bactériostatique et bactéricide, la lactoferrine présente dans le lait de femme protège le nourrisson des diarrhées infantiles.
Un effet antifongique de la lactoferrine humaine a été établi sur plusieurs souches de *Candida.* Des études ont également été réalisées avec la lactoferrine bovine et ont démontré cette action à la fois sur des levures et sur des champignons filamenteux. L'effet de la lactoferricine bovine serait d'ailleurs supérieur à celui de l'apolactoferrine bovine entière et similaire à celui de la polymyxine B, un antibiotique de type peptide cationique connu pour ses propriétés de destabilisation des membranes. Il a été également démontré que la lactoferricine B interagit directement à la surface du champignon en induisant ainsi des modifications de son ultrastructure.
Récemment, plusieurs auteurs ont mis en évidence une activité antivirale de la lactoferrine humaine. En effet, certains types de virus pénètrent dans la cellule par un mécanisme qui fait intervenir une adsorption des protéoglycannes des membranes des cellules cibles, suivie d'une fixation à un récepteur spécifique et par la fusion de la membrane virale avec celle de l'hôte. La lactoferrine se fixant aux héparanes sulfates des cellules grâce à sa forte basicité est ainsi capable d'inhiber l'adsorption de plusieurs types de virus. Des études *in vitro* très concluantes ont été réalisée avec le virus HIV (human immunodeficiency virus) et avec HCMV (human cytomegalovirus) avec une CI50 de l'ordre de 40µg/ml. Des résultats semblables ont été obtenus avec le virus HSV-1 (herpes simplex virus type 1). Ces derniers travaux ont mis en évidence non seulement un blocage des récepteurs du virus (héparanes sulfates, protéoglycannes, récepteur LDL) mais aussi une possible interaction entre la lactoferrine et le virus. Ces rôles récemment découverts représentent une nouvelle voie dans la prévention et la thérapie, en particulier pour les malades immunodéficients ou récidivistes vis à vis de ces infections virales.
Une des conséquences de l'inflammation d'un tissu est la formation de radicaux libres et la peroxydation des lipides. La formation de ces radicaux libres provient notamment des mécanismes de phagocytose de micro-organismes. Selon la réaction d'Haber-Weiss, les radicaux libres sont engendrés grâce au fer ferrique qui agit comme catalyseur de la réaction. Il a été démontré *in vivo* que la lactoferrine provenant de la dégranulation des neutrophiles stoppe la formation des radicaux libres extra-cellulaires en séquestrant immédiatement le fer qui catalyserait la formation de ces radicaux. Par cette action, la lactoferrine évite que les tissus ne soient endommagés. Par un mécanisme analogue, la lactoferrine inhibe la peroxydation des lipides et protège ainsi les membranes cellulaires sur lesquelles elle se fixe. Ces études ont ainsi démontré que la lactoferrine possède une activité anti-oxydante et anti-inflammatoire à la condition que la protéine soit sous sa forme privée de fer.
La lactoferrine libérée par les granules leucocytaires a été identifiée comme un inhibiteur de la production des colonies de granulocytes et de macrophages en diminuant la production de GM-CSF (facteur de stimulation des colonies de granulocytes et de macrophages). Le mécanisme mis en jeu semble complexe puisque la lactoferrine agirait en inhibant la libération d'une monokine responsable elle même de la libération du GM-CSF par les lymphocytes, les fibroblastes et les cellules endothéliales. Cette monokine a été identifiée à l'ILl.

De nombreux rôles de la lactoferrine, comme la suppression de la production d'anticorps, la régulation de l'activation du complément ou encore la régulation de l'activité des cellules NK (Natural Killer), impliquent des mécanismes qui sont régulés par des cytokines. Des études montrent que la lactoferrine peut exercer un rétrocontrôle négatif sur certaines cytokines comme l'IL1., l'IL2, et le TNFa afin d'empêcher la mobilisation et l'activation des leucocytes sur le lieu de l'inflammation.
Zimecki *et al*., (1991) ont démontré que des thymocytes immatures CD4- CD8- incubés en présence de lactoferrine acquièrent le marqueur CD4+ caractéristique des lymphocytes auxiliaires. Ces auteurs mettent également en évidence différents changements phénotypiques et fonctionnels de cellules B mis en présence de lactoferrine (Zimecki *et al*., *1995*). Un récepteur spécifique de la lactoferrine humaine a été caractérisé sur les lymphocytes activés (Mazurier *et al*., 1989). Par ailleurs, le site d'interaction de la lactoferrine avec son récepteur lymphocytaire a été décrit: Legrand *et al*. (1992) ont démontré qu'il était compris dans la région N terminale, et plus précisément dans les 50 premiers résidus de la lactoferrine.

La lactoferrine semble réguler l'activité cytotoxique des cellules NK (Natural Killer et des cellules LAK à des doses très faibles (0,75 µg/ml). La lactoferrine augmente de manière significative l'activité cytotoxique des cellules NK vis à vis des cellules tumorales et des cellules infectées par les rétrovirus. La lactoferrine agit aussi sur la cytotoxicité des monocytes. La cause de cette stimulation par la lactoferrine de l'activité cytotoxique des cellules NK, des lymphocytes mais aussi des cellules adhérentes peut être la conséquence, soit d'une activation des cellules tueuses à la suite de l'internalisation de la lactoferrine, soit d'une modification des cellules cibles qui deviendraient alors plus sensibles à la lyse.

La lactoferrine semble également jouer un rôle immunorégulateur dans la réponse inflammatoire (Elass-Rochard et al., 1995 ; confirmé par Elass-Rochard et al., 1998) ; la mise en évidence d'une activité antitumorale a également fait l'objet de travaux de la part de Salmon et al. (1998).

La lactoferrine exerce une activité facteur de croissance sur différentes cellules dans un milieu dépourvu de sérum de veau foetal. Cette activité a été mise en évidence notamment sur des lignées lymphocytaires humaines B et T et vis à vis d'une lignée murine macrophagique (P388 DI). La lactoferrine stimule également l'incorporation de thymidine tritiée dans l'ADN de cellules entérocytaires de rat.

Le rôle du fer dans l'activité facteur de croissance reste encore controversée: selon certains auteurs, la lactoferrine interviendrait en apportant le fer nécessaire à la prolifération cellulaire; pour d'autres, le fer ne jouerait aucun rôle, l'activité mitogène n'étant due qu'à la protéine elle-même.

L'hypothèse que la lactoferrine est impliquée dans l'absorption intestinale du fer est venue de l'observation que chez les nourrissons alimentés au lait maternel, l'incidence de la carence en fer est très faible. De même, seuls ces enfants maintiennent un important stock de fer jusqu'à l'âge de 6 mois, ce qui suggère une grande biodisponibilité du fer contenu dans le lait de femme. Ainsi, le pourcentage d'absorption peut atteindre 81% au cours des trois premiers mois de la vie puis diminue rapidement. Parmi les différents constituants du lait de femme, la lactoferrine est le meilleur candidat pour expliquer, d'une part, l'importante biodisponibilité du fer du lait et d'autre part, la régulation de cette absorption. Cox *et al*. ont étudié dès 1979 l'implication de la lactoferrine sur l'absorption du fer par l'intestin humain. Le récepteur entérocytaire de la lactoferrine a été mis en évidence pour la première fois chez le lapin, puis chez la souris. Enfin, le récepteur entérocytaire humain a été étudié et il a été montré sur des cultures de cellules entérocytaires HT29 que le nombre de récepteurs de lactoferrine était doublé en présence de chélateur de fer (Mikogami *et al,* 1994, 1995). Cette augmentation est due à une synthèse *de novo* de récepteurs. L'expression du récepteur de la lactoferrine est donc régulée par le manque de fer et cette carence induit également une augmentation de l'internalisation du récepteur de la lactoferrine par les entérocytes. Ainsi, le récepteur de la lactoferrine semble jouer un rôle dans la nutrition martiale en particulier en cas de carence en fer.

Outre la détermination de la séquence de l'ADNc de la lactoferrine humaine, la séquence nucléotidique de l'ADNc de la lactoferrine de différentes espèces animales a été établie. On peut notamment citer les séquences de :
- la lactoferrine bovine (Pierce et al, 1991),
- la lactoferrine porcine (Alexander B.F. et al, 1992),
- la lactoferrine murine (Shirsat N.V. et al, Gene, 1992),
- la lactoferrine caprine (Le Provost F. et al, 1994).

L'analyse des séquences peptidiques correspondant aux séquences des ADNc révèle l'existence d'homologies très importantes. En particulier, la séquence de la lactoferrine humaine présente 69% d'homologie avec celle de la vache. Comme dans la lactoferrrine humaine, la séquence nucléotidique de l'ADNc de la lactoferrine bovine renferme un peptide signal constitué de 16 acides aminés.
Cette homologie structurale entraîne des homologies fonctionnelles. En particulier, comme l'extrémité N-terminale de la lactoferrine bovine présente comme celle de la lactoferrine humaine un pourcentage élevé d'acides aminés basiques, ces deux lactoferrines sont reconnues d'une manière semblable par de nombreux constituants acides. Il faut noter par exemple une interaction semblable de ces deux protéines avec les lipopolysaccharides de bactéries Gram- et avec les protéoglycanes qui se trouvent localisés à la surface de nombreuses cellules.(Elass-Rochard E., et al., 1995).
Ces interactions semblables suggèrent que ces deux lactoferrines peuvent jouer un rôle identique dans l'inhibition de la libération des cytokines des macrophages activés par les lipopolysaccharides, donc dans les mécanismes des réactions inflammatoires.
De la même manière ces deux lactoferrines devraient montrer les mêmes propriétés de fixation sur les cellules entérocytaires et donc jouer un rôle identique dans l'absortion intestinale du fer et dans les différentes activités anti-bactériennes et anti-virales.

Ainsi, les nombreux rôles de la lactoferrine, et notamment ses activités antioxydantes, antimicrobiennes et antivirales donnent un intérêt majeur à cette protéine en terme de thérapie mais surtout de prophylaxie d'infections bactériennes, fongiques et virales mais aussi en ce qui concerne la prévention des chocs septiques survenants classiquement après une opération chirurgicale.

Les activités immunorégulatrices et antitumorales de la lactoferrine peuvent en outre être utilisées en thérapie inflammatoire et cancéreuse (Damiens et al. ; Zimecki et al.).
La lactoferrine peut également être utilisée dans une méthode de traitement dermopharmaceutique et cosmétique, par exemple dans un traitement cosmétique anti-radicaux libres ou pour protéger la kératine des cheveux contre les agressions atmosphériques.

### DESCRIPTION DETAILLE DE L'INVENTION:

L'invention concerne l'objet des revendications 1 à 19. Il est décrit:
- l'utilisation d'une séquence nucléotidique recombinante contenant, d'une part, un ADNc codant pour une lactoferrine, notamment une lactoferrine de mammifère, de préférence la lactoferrine bovine, porcine, caprine, humaine, ou les protéines dérivées (on entend par protéine dérivée toute protéine présentant au moins 70 % d'homologie avec la protéine de référence, notamment au moins 80%, par exemple entre 85 et 100% d'homologie et/ou présentant un profil de glycosylation différent tout en conservant les caractéristiques fonctionnelles de la lactoferrine de référence), et d'autre part, les éléments permettant à une cellule végétale de produire la lactoferrine ou les protéines dérivées, codées par ledit ADNc, notamment un promoteur et un terminateur de transcription reconnus par la machinerie transcriptionnelle des cellules végétales, pour la transformation de cellules de plantes en vue de l'obtention, à partir de ces cellules, ou de plantes obtenues à partir de ces dernières, de lactoferrine ou des protéines dérivées.
- une séquence nucléotidique recombinante, caractérisée en ce qu'elle contient d'une part la séquence codante pour une lactoferrine, notamment une lactoferrine de mammifère, de préférence la lactoferrine bovine, porcine, caprine, humaine, ou les protéines dérivées et d'autre part, les éléments permettant à une cellule végétale de produire la lactoferrine ou les protéines dérivées codées par ladite séquence, notamment un promoteur et un terminateur de transcription reconnus par la machinerie transcriptionnelle des cellules végétales. De manière avantageuse, la séquence nucléotidique contient une séquence codant pour un peptide signal responsable de la sécrétion des polypeptides recombinants
- un vecteur, notamment un plasmide, contenant une séquence nucléotidique telle que précédemment décrite insérée le cas échéant en un site non essentiel pour sa réplication.
- un hôte cellulaire, notamment toute bactérie telle que Agrobacterium tumefaciens, transformé par un vecteur tel que précédemment décrit,
- un procédé d'obtention de lactoferrine, notamment la lactoferrine humaine, ou des protéines dérivées, caractérisé en ce qu'il comprend :
- la transformation de cellules végétales, notamment à l'aide d'un hôte cellulaire tel que précédemment décrit, lui-même transformé par un vecteur tel que précédemment décrit, de manière à intégrer dans le génome de ces cellules une séquence recombinante telle que précédemment décrite,
- le cas échéant, l'obtention de plantes transformées à partir des cellules transformées susmentionnées,
- la récupération de la lactoferrine, notamment la lactoferrine humaine, ou des protéines dérivées recombinantes produits dans lesdites cellules ou plantes transformées susmentionnées, notamment par extraction, suivie, le.cas échéant, par une purification,
- une plante, un extrait de plante ou une partie de plante, notamment feuilles et/ou fruits et/ou semences et/ou cellules de plantes, transformés génétiquement, caractérisée en ce qu'elle contient une (ou plusieurs) séquence(s) nucléotidique(s) recombinante(s) telles que précédemment décrite(s), intégrée(s) de façon stable dans leur génome, ces plantes étant choisies notamment parmi le colza, le tabac, le mais, le pois, la tomate, la carotte, le blé, l'orge, la pomme de terre, le soja, le tournesol, la laitue, le riz, la luzerne, et la betterave,
- une lactoferrine, notamment une lactoferrine humaine, ou protéine dérivée caractérisée en ce qu'elle est obtenue selon le procédé décrit plus haut,
- une plante, un extrait de plante ou une partie de plantes, notamment feuilles et/ou fruits et/ou semences et/ou cellules de plantes, transformés génétiquement caractérisés en ce qu'ils contiennent une lactoferrine, notamment la lactoferrine humaine, ou une protéine dérivée, ces plantes étant choisies notamment parmi le colza, le tabac, le maïs, le pois, la tomate, la carotte, le blé, l'orge, la pomme de terre, le soja, le tournesol, la laitue, le riz, la luzerne, et la betterave.
- l'utilisation de plantes, extraits de plantes ou parties de plantes tels que décrits précédemment, et/ou de protéines (lactoferrine, notamment la lactoferrine humaine,
ou protéine dérivée) telles que décrites précédemment pour l'obtention de compositions pharmaceutiques, médicales, odontologiques, cosmétiques ou biotechnologiques,
- un Biomatériau ou une composition pharmaceutique, médicale, odontologique, cosmétique ou biotechnologique caractérisée en ce qu'elle comprend des plantes, extraits de plante, parties de plantes et/ou de protéines (lactoferrine, notamment la lactoferrine humaine, ou protéine dérivée) telles que décrites précédemment.

Une composition pharmaceutique comprend notamment toute composition constituant ou entrant dans la fabrication d'une composition permettant de prévenir ou de traiter une pathologie ou un symptôme d'origine bactérienne, fongique ou virale, une inflammation ou une pathologie ayant une composante inflammatoire, les chocs septiques, une pathologie liée à un phénomène de croissance cellulaire ou à une carence en fer comme l'anémie.

Une composition cosmétique comprend notamment toute composition constituant (ou entrant dans la fabrication de) un additif pour préparation (crèmes, pommades, fards, onguents).
Avantageusement, les séquences nucléotidiques recombinantes décrites plus haut contiennent une (ou plusieurs) séquence(s) codant pour un peptide responsable de l'adressage des polypeptides recombinants dans un compartiment déterminé de la cellule végétale, notamment dans le réticulum endoplasmique ou dans les vacuoles, ou bien même à l'extérieur de la cellule, dans la paroi pectocellulosique ou dans l'espace extracellulaire aussi appelé apoplasme.
Parmi les terminateurs de transcription susceptibles d'être utilisés pour la transformation de cellules de plantes, on peut citer le terminateur polyA 35S du virus de la mosaïque du chou-fleur (CaMV), ou le terminateur polyA NOS, qui correspond à la région en 3' non codante du gène de la nopaline synthase du plasmide Ti *d'Agrobacterium tumefaciens* souche à nopaline.

A ce titre, l'invention a pour objet toute séquence nucléotidique recombinante telle que décrite dans la revendication 9 contenant le terminateur polyA 35S du CaMV, ou le terminateur polyA NOS *d'Agrobacterium tumefaciens*.

Parmi les promoteurs de transcription susceptibles d'être utilisés pour la transformation de cellules de plantes dans le cadre de la présente invention, on peut citer:
- le promoteur 35S (P35S), ou avantageusement le promoteur constitutif double 35S (Pd35S) du CaMV, ces promoteurs permettant l'expression des polypeptides recombinants décrits ci-dessus dans l'ensemble de la plante obtenue à partir de cellules transformées décrites ci-dessus, et sont décrits dans l'article de Kay *et al*., *1987,*
- le promoteur PCRU du gène de la cruciférine de radis permettant l'expression des polypeptides recombinants décrits ci-dessus uniquement dans les semences (ou graines) de la plante obtenue à partir de cellules transformées telles que descrites ci-dessus, et décrit dans l'article de Depigny-This *et al.,* 1992 ;
- les promoteurs PGEA1 et PGEA6 correspondant à la région 5' non codante des gènes de la protéine de réserve de graines, GEAI et GEA6, respectivement, d'Arabidopsis thaliana (Gaubier et al., 1993), et permettant une expression spécifique dans les graines,
- le promoteur chimérique super-promoteur PSP (Ni M et al., 1995), constitué de la fusion d'une triple répétition d'un élément activateur transcriptionnel du promoteur du gène de l'octopine synthase d'Agrobacterium tumefaciens, d'un élément activateur transcriptionnel du promoteur du gène de mannopine synthase et du promoteur mannopine synthase d'Agrobacterium tumefaciens,
- le promoteur actine du riz suivi de l'intron actine de riz (PAR-IAR) contenu dans le plasmide pActl-F4 décrit par McElroy et al. (1991),
- le promoteur HMGW (High Molecular Weight Glutenine) de blé (Anderson O.D. et al, 1989),
- le promoteur du gène de γzéine de mais (Pyzéine) contenu dans le plasmide pγ63 décrit dans Reina et al. (1990), et permettant l'expression dans l'albumen des semences de maïs.
A ce titre, l'invention a pour objet toute séquence nucléotidique recombinante telle que décrite dans la revendication 10 contenant le promoteur 35S du CaMV, le promoteur constitutif double 35S (Pd35S) du CaMV, ou le promoteur PCRU du gène de la cruciférine de radis, ou les promoteurs PGEA1 ou PGEA6 d'Arabidopsis thaliana, ou le super-promoteur PSP d'Agrobacterium tumefaciens, ou le promoteur PAR-IAR du riz, le promoteur HMGW de blé ou le promoteur pγzéine du maïs.

Les séquences codant pour un peptide d'adressage utilisées dans le cadre de la présente invention, peuvent être d'origine végétale, humaine ou animale.

Parmi les séquences codant pour un peptide d'adressage d'origine végétale, on peut citer:
- la séquence nucléotidique de 69 nucléotides (indiquée dans les exemples qui suivent) codant pour le prépeptide (peptide signal) de 23 acides aminés de la sporamine A chez la patate douce, ce peptide signal permettant l'entrée des polypeptides recombinants décrits ci-dessus dans le système de sécrétion des cellules végétales transformées décrites ci-dessus (à savoir principalement dans le réticulum endoplasmique),
- la séquence nucléotidique de 42 nucléotides (indiquée dans les exemples qui suivent) codant pour le propeptide N-terminal d'adressage vacuolaire de 14 acides aminés de la sporamine A chez la patate douce, permettant l'accumulation des polypeptides recombinants de l'invention dans les vacuoles des cellules végétales transformées selon l'invention,
- la séquence nucléotidique de 111 nucléotides (indiquée dans les exemples qui suivent) codant pour le prépropeptide de 37 acides aminés de la sporamine A constitué de la partie N-terminale vers la partie C-terminale des 23 acides aminés du peptide signal susmentionné suivis par les 14 acides aminés du propeptide susmentionné, ce prépropeptide permettant l'entrée de polypeptides recombinants décrits ci-dessus dans le système de sécrétion et leur accumulation dans les vacuoles des cellules végétales transformées décrites ci-dessus les trois séquences susmentionnées étant décrites dans les articles de Murakami et *al.,* 1986, et Matsuoka et al, 1991,
- le propeptide carboxyterminal de la lectine d'orge décrit notamment dans les articles de Schroeder *et al, 1993*, et de Bednarek et al, 1991,
- et le PRS (Pathogenesis Related Protein, Cornelissen et al, 1986) permettant la sécrétion.

On peut également citer, parmi les séquences codant pour un peptide d'adressage, celle codant pour les peptides KDEL, SEKDEL et HDEL et permettant un adressage dans le réticulum endoplasmique. Cette divulgation porte également sur toute séquence nucléotidique recombinante telle que décrite ci-dessus, contenant une séquence codant pour tout ou partie d'un peptide d'adressage vacuolaire, notamment celui de la sporamine A de la patate douce, cette séquence codant pour un peptide d'adressage vacuolaire étant située, dans ladite séquence nucléotidique recombinante, entre la séquence codant pour un peptide signal et celle codant pour ledit ADNc ou sa séquence dérivée, de telle sorte que le premier acide aminé N-terminal du peptide d'adressage vacuolaire soit lié au dernier acide aminé C-terminal du peptide signal, et que le dernier acide aminé C-terminal dudit peptide d'adressage soit lié au premier acide aminé N-terminal du polypeptide codé par ledit ADNc ou sa séquence dérivée, dans la protéine codée par ladite séquence nucléotidique recombinante. Cette divulgation porte également sur toute

séquence nucléotidique recombinante telle que décrite ci-dessus, contenant une séquence codant pour tout ou partie d'un peptide d'adressage vacuolaire, notamment celui de la lectine d'orge, cette séquence codant pour un peptide d'adressage vacuolaire étant située, dans ladite séquence nucléotidique recombinante, en aval de la séquence codant pour ledit ADNc ou sa séquence dérivée, de telle sorte que le premier acide aminé N-terminal du peptide d'adressage vacuolaire soit lié au dernier acide aminé C-terminal du polypeptide codé par ledit ADNc ou sa séquence dérivée, dans la protéine codée par ladite séquence nucléotidique recombinante.

A ce titre, l'invention a pour objet toute séquence nucleotidique recombinante telle que décrite dans la revendication 7. **EXEMPLE 1: Construction du plasmide pBS-Lf renfermant l'ADNc complet codant la lactoferrine humaine.**

La lactoferrine est synthétisée sous la forme d'une pré-protéine dans laquelle la séquence codant pour la protéine mature dé 692 acides aminés est précédée d'un peptide signal de 19 acides aminés (PSLf), de séquence MKLVFLVLLFLGALGLCLA.

L'ADNc entier contenant la séquence de ce peptide signal, a été utilisé dans ce travail. Il a été isolé à partir d'une banque d'ADNc de glande mammaire humaine (Clontech, ref HL1037b, CA, USA) construite dans le vecteur λgt11. La banque a été criblée grâce à une sonde oligonucléotidique correspondant aux acides aminés du peptide signal (c'est-à-dire les acides aminés 1 à 19 de la hLf immature dite aussi prélactoferrine humaine) de la Lf par la technique d'hybridation sur réplique de nitrocellulose. Brièvement, les clones sont fixés dans un bain de NaOH 0,5M, NaCl 1,5M, pendant deux min avant d'être neutralisés dans une solution de NaCl 1,5 M, Tris-HCl 0,5 M, pH 7,4 durant 5 min. Les filtres sont alors rincés par du 2x SSC (17,5g/1 NaCl' 8,8g/1 citrate trisodique, QSP 11) . L'ADN est finalement fixé par cuisson 2h à 80°C L'hybridation a été réalisée à l'aide de la sonde oligonucléotidique décrite ci-dessus marquée au ³²p, à raison de 10⁶cpm/ml de solution d'hybridation (6X SSC, 10X Denhardt, 0,1% (p/v) nonidet NP40 (octyl phenoxypolyethoxyethanol, Sigma), 100,µg/ml d'ADN de sperme de saumon) durant une nuit, à 65°C. Les répliques sont alors lavées 4 fois dans un solution de 2X SSC, 0,1%SDS à 42°C, avant d'être placés en exposition.

Par hydrolyse ménagée de λgt11 par EcoRI (3 U/µg d'ADN pendant 2 min à 37°C), l'ADNc entier de la Lf a été cloné dans le plasmide pBluescript SK (Stratagene, La Jolla, USA) au site EcoRI, conduisant ainsi au plasmide pBS-Lf.

### EXEMPLE 2: Construction du plasmide pBS-12 renfermant l'ADNc complet codant la lactoferrine humaine modifié en son extrémité 3'.

Une modification en 3' de l'ADNc de la lactoferrine humaine a été opérée afin de pouvoir fusionner la séquence située en aval du codon stop avec le terminateur contenu dans pBIOC21, décrit ultérieurement. Par PCR, cette modification a permis de supprimer le site naturel de restriction EcoRI (GAATTC) en amont du codon stop, d'en créer un 9 paires de bases en aval, suivi d'un site XbaI (TCTAGA).

Cette PCR sur matrice pBS-Lf a été effectuée grâce au jeu d'oligodésoxynucléotides suivant:
oligo 5': 5' ATGACAACACTGAGTGTCTGGCC 3' (correspondant aux acides nucléiques 1991 à 2011, c'est-à-dire aux acides aminés 664 à 671 de la hLf immature)
oligo 3' : 5'CCG**TCTAGAGAATTC**GTTTTACTTCCTGAGGAGTTCAC 3', qui contient les sites de mutations (oligonucléotide chevauchant le codon stop, correspondant à l'acide aminé 711 de la hLf immature).

Conditions de PCR :

Les réactifs utilisés lors des différentes PCR sont fournis par Promega (Charbonnière, France), les oligodésoxynucléotides sont synthétisés par Eurogentec (Seraing, Belgique). Dans chaque cas, 5 ng de matrice sont incubés en présence de 100 pmol de chacun des deux oligodésoxynucléotides, 3 µl de dNTP 10 nM, 6 µl de MgCl₂ 25 mM, 0,5 , µl (2,5 U) de Taq DNA polymerase, dans un volume final de 100 , µl comprenant le tampon du fournisseur.

La PCR a été réalisée par un bio-med THERMOCYCLER 60 (B. Braun). La première dénaturation a eu lieu 5 min à 94°C. Elle a été suivie de 30 cycles comprenant chacun 1 min à 94 °C, 1 min à (Tm-10 °C soit 50°C) et 1 min à 72 °C. Pour terminer, une dernière phase d'élongation de 5 min à 72 °C a été effectuée.
Le produit issu de cette PCR contient le site unique NdeI (acide aminé 678 de la hLf immature) de l'ADNc de la lactoferrine . Ainsi, ce fragment a été hydrolysé par XbaI et NdeI et sous-cloné dans pBS-Lf délété du fragment NdeI-XbaI, ce qui a permis l'obtention du plasmide **pBS-12.**

Les conditions de ligation ont été les suivantes: 100ng de plasmides et 100ng d'inserts ont été incubés 1 nuit à 16°C, en présence de 4U de T4 DNA ligase (Stratagene), dans le tampon et les conditions préconisés par le fournisseur, soit dans un volume final de 50µl contenant entre autre 1µl d'ATP 10 mM. Ces conditions ont été utilisées pour toutes les réactions de ligation décrites ci-après.

Le produit issu de la ligation a permis la transformation de bactéries DH5α préalablement rendues compétentes par la technique au Rubididum décrite ci-dessous.

### Préparation des bactéries compétentes:

1 ml de culture de nuit de DH5α est remis en culture dans du milieu LB (10g/l de bactotryptone, (g/l de yeast extract, 5 g/l de NaCl) contenant du KCl (250 mM) et du MgSO4 (16 mM) jusqu'à l'obtention d'une DO₆₅₀ de 0,3. La suspension bactérienne est alors centrifugée à 2500 rpm pendant 5 min à 4°C ; le culot est repris dans du tampon Tfb1 (RbCl 100mM, MnCl₂ 50 mM, CaCl₂ 10mM, KOAc 10mM, glycérol 15%, pH5,8 ajusté avec HOAc 0,2M), incubé sur la glace durant 15 min puis centrifugé de nouveau dans les mêmes conditions. Le culot est cette fois repris par 6,5 ml de tampon Tfb2 (MOPS 10 mM, RbCl 10 mM, CaCl₂ 75 mM, Glycerol 15%, pH7 ajusté avec NaOH 0,5M). Les cellules sont alors réparties à raison de 200 µl par tube et stockées à -80°C pour les étapes ultérieures de transformation.

Lors de l'étape de transformation, les cellules sont décongelées sur la glace, la solution d'ADN (25 µl de la réaction de ligation, soit 50 ng d'ADN) est ajoutée à 200 µl de DH5α compétentes. La suspension est incubée 20 min sur la glace puis plongée dans un bain-marie à 42°C pendant 90 sec et replacée sur la glace durant 5 min. La suspension est remise en culture liquide dans du milieu LB pendant 1 h, puis étalée sur milieu LB contenant le marqueur de sélection. Après une culture d'une nuit à 37°C, les clones sont analysés.

Cette technique a été utilisée pour toutes les transformations ultérieures.

Après analyse des clones de pBS12, la séquence du fragment amplifié par PCR est confirmée par séquençage (Sanger et *al.,* 1975) de ce plasmide (Sequenase DNA sequencing kit, United States Biochemical Corporation, Cleveland, USA).

### EXEMPLE 3: Construction du plasmide pBS-14 dérivé de pBS-12 renfermant l'ADNc codant la lactoferrine humaine dont la séquence du peptide signal (PSLf) a été remplacée par celle du peptide signal de la sporamine de patate douce (PSSp).

Premièrement, par PCR, un site SalI a été introduit en aval de XhoI dans l'ADNc de la Lf, au niveau du premier codon de la séquence de la protéine mature, afin de pouvoir fusionner avec la séquence codant le peptide signal de la sporamine de patate douce (PSSp; Murakami et al., 1986; Matsuoka et al., 1991) permettant théoriquement la sécrétion. Le peptide signal de la sporamine de patate douce est constitué de 23 acides aminés de séquence: MKAFTLALFLALSLYLLPNPAHS.

Les oligodésoxynucléotides, oligo 5':
5' TAA**CTCGAG**GCCGG**GTCGAC**GGAGAAGGAGTGTTCAGTG 3' contenant SalI (GTCGAC) et XhoI (CTCGAG); acides aminés 16 à 28 de la hLf immature,
et,
oligo3': 5' ACCCGT**CCAATTCAAGAATGG**ACGAAG 3' contenant XcmI (CCAATTCAAGAATGG, acide aminé 153 de la hLf immature), ont été utilisés pour cette PCR sur matrice pBS-12 dont les conditions sont décrites précédemment. Le fragment issu de PCR a été hydrolysé par XhoI et XcmI . Il a été inséré aux sites XhoI et XcmI de pBS-12 en mettant en oeuvre les conditions de ligation décrites précédemment. La transformation a été réalisée comme décrite précédemment. Le plasmide résultant est appelé pBS-13. Sa séquence a été vérifiée par séquençage comme décrit précédemment.

Deuxièmement, afin de fusionner l'ADNc du peptide signal de la sporamine à celui de la Lf mature, un site SalI (GTCGAC) a été introduit par PCR au niveau du dernier codon de la séquence du peptide de la sporamine, ainsi qu'un site XhoI (CTCGAG) suivi d'un site EcoRI immédiatement en amont du codon d'initiation ATG. Ces modifications ont été réalisées par PCR, sur matrice pMAT103 selon les conditions décrites ci-dessus. Les oligodéoxynucléotides choisis ont permis l'insertion de ces différents sites de restriction:
oligo 5': 5' TCC**CTCGAG**GAATTCATGAAAGCCTTCACACTC 3',
et,
oligo 3': 5' TCC**GTCGAC**CGGAATGGGCTGGATTGGGCAGG 3'.

Après digestion du fragment amplifié par XhoI et Sal L celui-ci a été sous-cloné dans pBS-13 préalablement hydrolysé par XhoI et SalI. Le plasmide résultant pBS-14 a été vérifié par séquençage des 500 premiers nucléotides de la protéine chimérique.

### EXEMPLE 4: Construction de pBIOC21.

L'expression dans les feuilles et les graines de tabac de l'ADNc codant la lactoferrine humaine a nécessité les séquences régulatrices suivantes :
1. le promoteur constitutif double 35S (Pd35S) du CaMV (virus de la mosaïque du chou-fleur). Il correspond à une duplication des séquences activant la transcription situées en amont de l'élément TATA du promoteur 35S naturel (Kay *et al*., 1987);
2. la séquence terminatrice de transcription, terminateur polyA 35S (T35S), qui correspond à la région 3' non codante de la séquence du virus à ADN bicaténaire circulaire de la mosaïque du chou-fleur produisant le transcrit 35S (Franck et *al.,* 1980).

Les constructions des différents plasmides via l'utilisation des techniques d'ADN recombinant dérivent de pBIOC4. Le plasmide binaire dérive de pGA492 (An, 1986). Le plasmide dérivant de pBIOC4 et contenant la cassette d'expression "PD35S-T35S" est le plasmide pBIOC21.

Les différents éléments permettant de reproduire ces constructions sont par exemple contenus dans la description de la demande de brevet W09633277 qui est incorporée par référence.

### EXEMPLE 5: Construction de pBIOC21-PSLf-Lf.

Le plasmide pBS-12 a été hydrolysé par EcoRI pour isoler le fragment de 2160 paires de bases correspondant à la séquence codant la lactoferrine humaine précédée de son peptide signal naturel. Ce fragment a été sous-cloné dans le plasmide binaire pBIOC21 préalablement linéarisé en EcoRI et déphosphorylé. Le plasmide binaire obtenu est appelé pBI0C21-PSLf-Lf.

La réaction de déphosphorylation est appliquée à 800 ng d'ADN en présence de 1U de CIP (Stratagene) dans le tampon du fournisseur, dans un volume de 40, µl pendant 30 min à 37°C. Les réactions de ligation et de transformation de la souche DH5α *d'Escherichia coli* ont été réalisées comme mentionné ci-dessus.

L'ADN plasmidique de pBIOC2-1-PSLf-Lf a été introduit dans la souche LBA4404 *d'Agrobacterium tumefaciens* selon le procédé de Holsters (1978). La validité des clones retenus est vérifiée par digestion enzymatique de l'ADN plasmidique introduit.

### EXEMPLE 6: Construction de pBIOC21-PSSp-Lf.

L'ADNc codant la protéine chimérique "PSSp-Lf" a été excisé de pBS-14 par hydrolyse par EcoRI. Ce fragment codant de 2150 paires de bases a été sous-cloné au site EcoRI déphosphorylé de pBIOC21. Le plasmide binaire obtenu est appelé pBIOC21-PSSp-Lf.

Les réactions de déphosphorylation, ligation, transformation de la souche DH5α *d'Escherichia coli* ont été réalisées comme mentionné ci-dessus.

L'ADN plasmidique de pBIOC21-PSSp-Lf a été introduit dans la souche LBA4404 *d'Agrobacterium tumefaciens* selon le procédé de Holsters (1978). La validité des clones retenus est vérifiée par digestion enzymatique de l'ADN plasmidique introduit.

### EXEMPLE 7 :Construction de pACT-IA-PSLf-Lf

L'expression constitutive dans les semences de maïs a nécessité les séquences régulatrices suivantes:
- promoteur actine de riz suivi de l'intron actine de riz (pAR-IAR) contenu dans le plasmide pAct1 -F4 décrit par McElroy *et al.* (1991);
- la séquence terminatrice de transcription, terminateur polyA NOS, qui correspond à la région en 3' non codante du gène de la nopaline synthase du plasmide Ti *d'Agrobacterium tumefaciens* souche à nopaline (Depicker et al., 1982).

Le plasmide pBSII-pAR-IAR-tNOS dans lequel a été inséré la séquence codant "PSLf- Lf" est décrit par exemple dans la demande de brevet W09633277 qui est incorporée par référence.

La séquence codant "PSLf-Lf" a été isolée par digestion enzymatique EcoRI à partir de pBS-12. Le fragment digéré a été purifié par électrophorèse sur gel d'agarose à 0.8%, puis soumis à l'électroélution, à la précipitation alcoolique, séché, repris dans H₂0 Il a été traité par action de l'enzyme Klenow (New England Biolabs) selon les recommandations du fabricant. Il a été inséré dans le plasmide pBSII-pAR-IAR-tNOS digéré doublement par SalI et NcoI, purifié, traité à l'enzyme Mung Bean Nucléase (New England Biolabs) et déphosphorylé par l'enzyme phosphatase alcaline de veau (Boehringer Mannheim) selon les recommandations des fabricants. La ligation a été réalisée avec 20 ng du vecteur déphosphorylé et 200 ng de fragments d'ADN contenant la séquence codant pour "PSLf-Lf", décrits ci-dessus, dans un milieu réactionnel de 20 µl en présence de 2 µl de tampon T4 DNA ligase x 10 (Amersham), de 2 µl de 50% polyethylène glycol 8000 et de 5 U d'enzyme T4 DNA ligase (Amersham) à 14°C pendant 16 heures. Les bactéries, *Escherichia coli* DH5α rendues préalablement compétentes, ont été transformées (Hanahan, 1983). L'ADN plasmidique des clones obtenus, sélectionnés sur 50 µg/ml ampicilline, a été extrait selon la méthode de la lyse alcaline (Birnboim et Doly, 1979) et analysé par digestion enzymatique par des enzymes de restriction. Le plasmide résultant a été appelé pACT- IA-PSLf-Lf.

### EXEMPLE 8: Construction de pACT-IA-PSSp-Lf

La construction pACT-IA-PSSp-Lf est similaire à pACT-IA-PSLf-Lf excepté que la séquence "PSLf-Lf" a été remplacée par celle codant "PSSp-Lf" correspondant au fragment EcoRI traité par l'enzyme Klenow (New England Biolabs), isolé à partir de pBS-14. Le plasmide résultant est appelé pACT-IA-PSSp-Lf.

### EXEMPLE 9: Construction de pgzéine-PSLf-Lf

L'expression dans l'albumen des semences de maïs a nécessité les séquences régulatrices suivantes:
- le promoteur du gène gamma (g) de zéine de maïs(Pgzéine) contenu dans le plasmide p63 décrit dans Reina et al., 1990. Le plasmide p63 résulte du clonage de Pgzéine, en remplacement du promoteur 35S (P35S), aux sites HindIII et XbaI du plasmide pUC18 renfermant, entre ses sites HindIII et EcoRI, la cassette d'expression "P35S-gus-TNOS" de pBI221 commercialisé par Clontech. Il permet une expression dans l'albumen des semences maïs.
- la séquence terminatrice de transcription, terminateur polyA NOS, qui correspond à la région en 3' non codante du gène de la nopaline synthase du plasmide Ti *d'Agrobacterium tumefaciens* souche à nopaline (Depicker *et al*., 1982).

Le plasmide pgzéine-PSLf-Lf où la séquence "PSLf-Lf' est placée sous contrôle du Pgzéine, a été obtenu par clonage aux sites, SacI et BamHI, traités par l'enzyme T4 DNA polymérase (New England Biolabs), puis déphosphorylés par l'enzyme phosphatase alcaline de veau (Boehringer Mannheim) du plasmide p63, du fragment EcoRI traité à la Klenow (New England Biolabs) isolé à partir de pBS-12. La ligation a été réalisée comme décrite dans l'exemple 7. Les bactéries, *Escherichia coli* DH5α rendues préalablement compétentes, ont été transformées (Hanahan, 1983). L'ADN plasmidique des clones obtenus, sélectionnés sur 50 µg/ml ampicilline, a été extrait selon la méthode de la lyse alcaline et analysé par digestion enzymatique par des enzymes de restriction. Le clone résultant a été appelé pgzéine-PSLf-Lf.

### EXEMPLE 10: Construction de pgzéine-PSSp-Lf

La construction pgzéine-PSSp-Lf est similaire à pgzéine-PSLf-Lf excepté que la séquence codant "PSL.f-Lf" a été remplacée par celle codant "PSSp-Lf' correspondant au fragment EcoRI traité par l'enzyme Klenow (New England Biolabs), isolé à partir de pBS-14. Le plasmide résultant est appelé pgzéine-PSSp-Lf.

### EXEMPLE 11 : Construction de pHMWG-IA-PSLf-Lf.

L'expression dans les semences de maïs a nécessité les séquences suivantes :
- le promoteur du gène HMWG (high-molecular-weight glutein) de blé (Anderson et al 1989) suivi de l'intron actine de riz,
- la séquence terminatrice de transcription, terminateur poly-A35S (T35S), qui correspond à la région 3' non codante de la séquence du virus à ADN bicaténaire circulaire de la mosaïque du chou-fleur produisant le transcrit 35S (Franck et al., 1980).
Le plasmide pHMWG -IA résulte du clonage de PHMWG-IA en remplacement du promoteur PD35S (P35S) du plasmide pJIT163. Les différents éléments permettant de reproduire cette construction sont notamment contenus dans la description de la demande de brevet internationale No.WO 96/33277.

Le plasmide pHMWG-IA-PSLf-Lf où la séquence « PSLf-Lf » est placée sous contrôle du PHMWG-IA, a été obtenu par clonage au site EcorI déphosphorylé par l'enzyme phophatase alcaline de veau (Boehringer Manheim) du plasmide pHMWG-IA, du fragment EcorI isolé à partir de pBS-12. La ligation a été réalisée avec 100 ng du vecteur déphosphorylé et 50 ng de fragments d'ADN contenant la séquence codant pour « PSLf-Lf », décrits ci-dessus, dans un milieu réactionnel de 10 µl en présence de 1 µl de tampon T4DNA ligase (Amersham) à 14°C pendant 16 heures. Les bactéries, *Escherichia coli* DH5α rendues préalablement compétentes, ont été transformées (Hanahan, 1983). L'ADN plasmidique des clones obtenus, sélectionnés sur 50 µg/ml ampicilline, a été extrait selon la méthode de la lyse alcaline et analysé par digestion enzymatique par des enzymes de restriction. Le clone résultant a été appelé **pHMWG-IA-PSLf-Lf.**

### EXEMPLE 12 : Construction de pHMWG-IA-PSSp-Lf.

La construction de pHMWG-IA-PSSp-Lf est similaire à celle de pHMWG-IA-PSLf-Lf excepté le fait que la séquence codant pour « PSLf-Lf » a été remplacée par celle codant pour « PSSp-Lf » correspondant au fragment EcoRI isolé à partir de pBS14. Le plasmide résultant est appelé **pHMWG-IA-PSSp-Lf.**

### EXEMPLE 13 : Obtention de plants de solanacees transgeniques.

### A. Transformation des plantes de tabac.

Les plants de tabac utilisées pour les expériences de transformation *(Nicotiana tabacum* var. *Xanthi* NC) sont cultivées *in vitro* sur le milieu de base de Murashige et Skoog (1962) additionné des vitamines de Gamborg *et al*. (1968, Sigma référence M0404) de saccharose à 20 g/l et d'agar (Merck) à 8 g/l. Le pH du milieu est ajusté à 5,8 avec une solution de potasse avant autoclave à 120°C pendant 20 min. Les plantules de tabac sont repiquées par bouture des entre-noeuds tous les 30 jours sur ce milieu de multiplication MS20 (M0404 à 4,4 g/1, saccharose à 20 g/1, agar agar à 8 g/1, pH5,7).

Toutes les cultures *in vitro* sont réalisées en enceinte climatisée, dans les conditions définies ci-dessous:
- intensité lumineuse de 30 µE m⁻².S⁻¹; photopériode de 16h;
- thermopériode de 26°C le jour, 24°C la nuit.

La technique de transformation utilisée est dérivée de celle de Horsch *et al*. (1985).

Une préculture *d'Agrobacterium tumefaciens* souche LBA4404 contenant les plasmides binaires est réalisée durant 48h à 28°C sous agitation, dans du milieu LB additionné des antibiotiques adéquats (rifampicine et tétracycline). La préculture est ensuite diluée au 50ème dans le même milieu et cultivée dans les mêmes conditions.

Après une nuit, la culture est centrifugée (10 min., 3000 g), les bactéries sont reprises dans un volume équivalent de milieu MS30 liquide (M0404 à 4,4 g/l, saccharose à 30 g/l, pH5,7) et cette suspension est diluée au 10ème.

Des explants d'environ 1 cm² sont découpés à partir des feuilles des plantules décrites ci-dessus. Ils sont ensuite mis au contact de la suspension bactérienne pendant 1h, puis séchés rapidement sur papier filtre et placés sur un milieu de coculture (MS30 solide correspond à MS30 liquide additionné d'agar agar à 8 g/l, de BAP 1 mgA et d'ANA 0,1 mg/l).

Après 2 jours, les explants sont transférés en boites de Pétri sur le milieu de régénération MS30 (correspondant au milieu de coculture supplémenté en kanamycine à 200 mgA et augmentin à 400 mgA). Ce milieu contient un agent sélectif, la kanamycine (200 mg/1), un bactériostatique, l'augmentin (400 mg/1) et les hormones nécessaires à l'induction de bourgeons (BAP, 1 mg/l et ANA, 0,1 mg/1). Un repiquage des explants est effectué sur le même milieu après 2 semaines de culture. Après 2 semaines supplémentaires, les bourgeons sont repiqués en boîtes de Pétri sur le milieu de développement composé du milieu MS20 additionné de kanamycine et d'augmentin. Après 15 jours, les bourgeons sont repiqués de moitié. L'enracinement prend environ 20 jours, au terme desquels les plantules peuvent être clonées par bouture d'entre-noeuds ou sorties en serre.

### B. Obtention de plantes de tomate transgéniques.

Les graines de tomate cv. UC82B sont stérilisées au Domestos 10% 15 min. et rincées 3 fois dans de l'eau stérile. Le dernier rinçage est effectué pendant 10 min. sous agitation.

Les graines ainsi stérilisées sont mises à germer sur milieu MSSV/2 (milieu de base de Murashige et Skoog (1962, Sigma référence M6899)/2 additionné des vitamines de Nitsch (Thomas et Pratt, 1981), de saccharose à 30 g/l, d'agar (Merck) à 8 g/l, pH 5,9, pendant 7 ou 8 jours en chambre climatisée (intensité lumineuse de 30 µE. m⁻², S⁻¹, photopériode de 16 h/8 h, 26°C).

La technique de transformation utilisée est dérivée de celle de Fillatti *et al*. *(1987).*

Une préculture *d'Agrobacterium tumefaciens* souche LBA4404 contenant les plasmides binaires est réalisée pendant 24 h à 28°C sous agitation dans du milieu LB additionné des antibiotiques adéquats (rifampicine et tétracycline). La préculture est ensuite diluée au 50ème dans le même milieu et cultivée dans les mêmes conditions pendant une nuit La DO à 600 nm est mesurée, les agrobactéries sont centrifugées (10 min, 3000 g) et repris dans du milieu KCMS liquide (décrit dans la publication de Fillatti et *al.,* 1987) de manière à obtenir une DO à 600 nm de 0,8.

Des améliorations techniques ont été apportées à certaines étapes du protocole de Fillatti et al (1987). La préculture des explants et la coculture sont réalisées comme décrit par Fillatti *et al*. (1987) excepté que le milieu KCMS est supplémenté en acétosyringone (200 mM).

Le milieu de lavage 2Z diffère par l'addition de céfotaxime à 500 mg/l au lieu de carbénicilline. Le milieu de développement utilisé est composé du milieu de base de Murashige et Skoog (Sigma MS6899) additionné des vitamines de Nitsch, de saccharose à 20 g/l, de kanamycine à 50 mg/l, d'augmentin à 200 mg/l, d'ANA à 1 mg/l et de zéatine à 0,5 mg/l.

### EXEMPLE 14: Obtention de plants de colza transgeniques.

Les graines de colza de printemps *(Brassica napus* cv WESTAR ou lignées Limagrain) sont désinfectées pendant 40 minutes dans une solution de Domestos à 15%. Après 4 rinçages à l'eau stérile, les graines sont mises à germer, à raison de 20 graines par pot de 7 cm de diamètre et 10 cm de haut, sur du milieu minéral de Murashige et Skoog (Sigma M 5519) avec 30g/l de saccharose et solidifié avec de l'agargel à 5g/1. Ces pots sont placés dans une chambre de culture à 26°C avec une photopériode de 16h/8h et sous une intensité lumineuse de l'ordre de 80 µE m⁻² s⁻¹.

Après 5 jours de germination, les cotylédons sont prélevés stérilement en coupant chaque pétiole environ 1 mm au-dessus du noeud cotylédonaire.

Parallèlement une préculture *d'Agrobacterium tumefaciens* souche LBA4404, contenant le plasmide binaire, est réalisée en erlenmeyer de 50 ml, pendant 36 h à 28°C dans 10 ml de milieu bactérien 2YT complémenté avec les antibiotiques utiles à la sélection de la souche utilisée.

Cette préculture sert à ensemencer à 1% une nouvelle culture bactérienne effectuée dans les mêmes conditions. Au bout de 14 h la culture est centrifugée 15 min à 3000 g et les bactéries sont reprises dans un volume équivalent de milieu de germination liquide. Cette suspension est distribuée dans des boites de pétri de 5 cm de diamètre à raison de 5 ml/boite.

L'extrémité sectionnée du pétiole est immergée quelques secondes dans la solution d'agrobactéries ainsi préparées, puis le pétiole est enfoncé de quelques millimètres dans le milieu de régénération. Ce milieu a la même composition de base que le milieu de germination avec en plus 4 mg/l de benzyl-amino-purine (BAP), phytohormone favorisant la néoformation de bourgeons. Dix explants (cotylédon avec pétiole) sont mis en culture par boîte de pétri de 9 cm de diamètre (Greiner référence 664102).

Après 2 jours de coculture dans les mêmes conditions environnementales que les germinations, les explants sont repiqués dans des boîtes phytatray (Sigma, référence P1552), contenant le milieu précédent complémenté avec un agent sélectif: 45 mg/l de sulfate de kanamycine (Sigma, référence K4000) et un bactériostatique: mélange de 1/6(en poids) de sel de potassium d'acide clavulanique et de 5/6 sel de sodium d'amoxicilline (Augmentin injectable) à raison de 600 mg/l.

Deux fois de suite, à 3 semaines d'intervalle, les explants sont repiqués stérilement sur du milieu neuf dans les mêmes conditions.

Les bourgeons verts apparus à la fin du deuxième ou du troisième repiquage sont séparés de l'explant et mis en culture individuellement dans des pots transparents de 5 cm de diamètre et de 10 cm de haut contenant un milieu identique au précédent mais dépourvu de BAP. Après 3 semaines de culture la tige du bourgeon transformé est sectionnée et le bourgeon est repiqué dans un pot de milieu frais. Au bout de trois à quatre semaines les racines sont assez développées pour permettre l'acclimatation de la plantule dans un phytotron. Les bourgeons qui ne sont pas verts ou enracinés sont éliminés Ces plantules sont alors transplantées dans des godets de 7 cm de côté remplis de terreau (norme NF U4455 1: 40% tourbe brune, 30% bruyère tamisée et 30% sable) saturé en eau. Après deux semaines d'acclimatation en phytotron (température 21°C, photopériode 16h/8h et 84% d'humidité relative), les plantules sont rempotées dans des pots de 12 cm de diamètre remplis du même terreau enrichi en engrais retard (Osmocote, à raison de 4 g/l de terreau) puis transportées en serre (classe S2) régulée à 18°C, avec deux arrosages quotidien de 2 minutes à l'eau.

Dès l'apparition de fleurs celles-ci sont ensachées (Crispac, référence SM 570y 300 mm*700 mm) de façon à empêcher la fécondation croisée.

Lorsque les siliques sont arrivées à maturité, elles sont récoltées, séchées, puis battues. Les graines obtenues servent au dosage de l'activité biochimique. La sélection de la descendance transgénique se fait par germination sur un milieu contenant du sulfate de kanamycine à raison de 100 à 150 mg/l (selon les génotypes). Les conditions opératoires sont identiques à celles décrites ci-dessus à ceci près que les germinations sont effectuées en tubes de verre avec une seule graine par tube. Seules les plantules développant des racines secondaires les trois premières semaines sont acclimatées en phytotron avant d'être passées en serre.

### EXEMPLE 15: Obtention de plantes de maïs transgeniques.

### A. Obtention et utilisation de cal de maïs comme cible pour la transformation génétique.

La transformation génétique du maïs, quelle que soit la méthode employée (électroporation; Agrobacterium, microfibres, canon à particules), requiert généralement l'utilisation de cellules indifférenciées en divisions rapides ayant conservé une aptitude à la régénération de plantes entières. Ce type de cellules compose le cal friable embryogène (dit de type II) de maïs.
Ces cals sont obtenus à partir d'embryons immatures de génotype Hl II ou (A188 x B73) selon la méthode et sur les milieux décrits par Armstrong (1994) Les cals ainsi obtenus sont multipliés et maintenus par repiquages successifs tous les quinze jours sur le milieu d'initiation.

Des plantules sont ensuite régénérées à partir de ces cals en modifiant l'équilibre hormonal et osmotique des cellules selon la méthode décrite par Vain et al. (1989). Ces plantes sont ensuite acclimatées en serre où elles peuvent être croisées ou autofécondées.

### B. Utilisation du canon à particules pour la transformation génétique du maïs.

Le paragraphe précédent décrit l'obtention et la régénération des lignées cellulaires nécessaires à la transformation; on décrit ici une méthode de transformation génétique conduisant à l'intégration stable des gènes modifiés dans le génome de la plante. Cette méthode repose sur l'utilisation d'un canon à particules identique à celui décrit par J. Finer (1993) ; les cellules cibles sont des fragments de cals décrits dans le paragraphe 1 Ces fragments d'une surface de 10 à 20 mm² ont été disposés, 4 h avant bombardement, à raison de 16 fragments par boîte au centre d'un boîte de petri contenant un milieu de culture identique au milieu d'initiation, additionné de 0,2 M de mannitol + 0,2 M de sorbitol. Les plasmides portant les gènes à introduire sont purifiés sur colonne Qiagen" en suivant les instructions du fabricant. Ils sont ensuite précipités sur des particules de tungsten (M10) en suivant le protocole décrit par Klein (1987). Les particules ainsi enrobées sont projetées vers les cellules cibles à l'aide du canon et selon le protocole décrit par J. Finer (1992).

Les boites de cals ainsi bombardées sont ensuite scellées à l'aide de Scellofrais, puis cultivées à l'obscurité à 27°C. Le premier repiquage a lieu 24 h après, puis tous les quinze jours pendant 3 mois sur milieu identique au milieu d'initiation additionné d'un agent sélectif dont la nature et la concentration peuvent varier selon le gène utilisé (voir paragraphe 3). Les agents sélectifs utilisables consistent généralement en composés actifs de certains herbicides (Basta ™, Round up™,) ou certains antibiotiques (Hygromycine, Kanamycine...).

On obtient après 3 mois ou parfois plus tôt, des cals dont la croissance n'est pas inhibée par l'agent de sélection, habituellement et majoritairement composés de cellules résultant de la division d'une cellule ayant intégré dans son patrimoine génétique une ou plusieurs copies du gène de sélection. La fréquence d'obtention de tels cals est d'environ 0,8 cal par boîte bombardée

Ces cals sont identifiés, individualisés, amplifiés puis cultivés de façon à régénérer des plantules (cf. paragraphe A exemple 15). Afin d'éviter toute interférence avec des cellules non transformées toutes ces opérations sont menées sur des milieux de culture contenant l'agent sélectif.

Les plantes ainsi régénérées sont acclimatées puis cultivées en serre où elles peuvent être croisées ou autofécondées.

Les plantes obtenues selon l'invention possèdent un phénotype distant de celui obtenu dans W09637094.

### EXEMPLE 16-a : Détection des lactoferrines humaines recombinantes (rhLf) à partir de feuilles de plante.

Afin de tester les plantes les plus productives, une extraction des protéines solubles a été réalisée sur une vingtaine d'événements de transformation pour chacune des constructions pBIOC21-PSLf-Lf et pBIOC21-PSSp-Lf, suivie d'une mise en évidence qualitative et quantitative de la Lf recombinante (rhLf). Ainsi, les feuilles ont été broyées dans l'azote liquide jusqu'à l'obtention d'une poudre fine. Les protéines solubles ont été extraites à partir de la matière végétale par un tampon d'extraction (Tris-HCl 50 mM, EDTA 1 mM, NaCl 100 rnM, Triton X 100 0.1 %, pH6,5), à raison de 4 ml de tampon par gramme de feuille. Après centrifugation de l'extrait pendant 30 min à 13 000 g, le surnageant a été décanté, filtré et utilisé pour les étapes ultérieures de mise en évidence de la lactoferrine recombinante.

Méthode ELISA de détection de la Lactoferrine (Micogami et al, 1994*).*

La sélection d'un transformant pour chaque type de construction moléculaire se fait en comparant la quantité de Lf recombinante à la quantité de protéines solubles. Le dosage des protéines solubles a été réalisé par microtitration (Promega, Charbonnière, France), et le taux d'expression de rhLf a été mesuré par technique ELISA décrite ci-dessous.

Un anticorps polyclonal de lapin anti-lactoferrine humaine a été produit comme suit: L'immunisation du lapin a été réalisée par 3 injections intra-musculaires successives de hLf. Lors de la première injection, 1 mg de hLf dissoute dans 0,25 ml de sérum physiologique apyrogène et 0,25 ml d'adjuvant complet de Freund (Difco) ont été injectés. Après 2 semaines, une seconde injection a été réalisée avec 1 mg de hLf dissoute dans 0,25 ml de sérum physiologique apvrogène et 0,25 ml d'adjuvant imcomplet de Freund (Difco). Puis, au bout de 4 semaines, une troisième injection a été réalisée dans les mêmes conditions que la seconde. Un premier prélèvement sanguin effectué 10 à 15 jours après la troisième injection a permis d'obtenir 20 ml de sang. Puis, des injections correspondant à des rappels d'immunisation (même condition que seconde injection décrite ci-dessus) ont permis des prélèvements répétés tous les 10 jours. Les immunoglobulines de l'antisérum de lapin ont été purifiées par précipitation au sulfate d'ammonium à 35% de saturation. Le précipité a été dialysé dans un tampon TrisHCI 10 mM pH8, puis purifiés par chromatographie sur DEAE Trisacryl (IBF).

Les anticorps polyclonaux ont été incubés dans les puits de la plaque de microtitration à raison de 100 µl par puits (soit 35 µg /ml) dans un tampon bicarbonate de sodium (NaHCO3) 10 mM (pH 9,6) une nuit à 4°C ou 2h à 37 °C.

Une incubation de la plaque ELISA (Falcon) dans 150 µl de PBS (NaCl 150 mM, phosphate de sodium (Na2HPO4, NaH2PO4) 50 mM, pH7,5) - tween 2% pendant 20 min à température anmbiante a permis de bloquer les sites non spécifiques. Après trois lavages au PBS - tween 0,05%, 100 µl de chaque échantillon issus des extraits de protéines solubles ont été incubés à 37 °C pendant 2h avant que la hLf ne soit détectée par des Ac monoclonaux anti-Lf. Les Ac monoclonaux anti-Lf ont été produits par fusion de splénocytes de souris et de cellules de myélome SP₂O/Ag. Le surnageant du milieu de culture de ces hybridomes a été utilisé directement lors du test ELISA. Le complexe Ac-rhLf a été reconnu par des anticorps de chèvre anti-souris marqués à la péroxydase (Diagnostic Pasteur) dilué au 1/3000e dans du PBS et a été mis en contact, comme précédemment, 2h à 37 °C. La révélation est réalisée sous agitation par addition de la solution de substrat (4 mg d'ortho-phenylénédiamine dihydrochloride dans 10 µl de tampon " acide citrique / citrate de sodium " 0,2M pH 5,5 en présence de 10 µl d'H₂O₂), la coloration a été obtenue en quelques minutes, et la réaction a été stoppée par ajout de 50 µl par puits de H₂SO₄ à 20 %. La densité optique a été lue à 490 mM.

Entre chaque étape, les puits ont été lavés au PBS-tween 0,05 %.

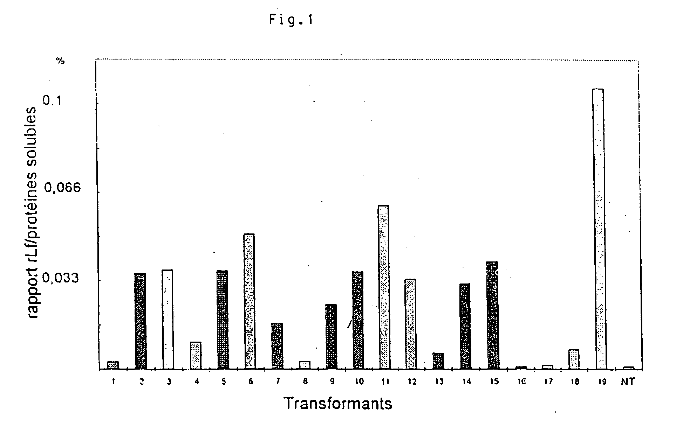

Le rapport rhLf sur protéines solubles est représenté dans le diagramme Fig. 1. Il a permis de sélectionner le transformant T19 pour la construction pBIOC21-PSLfLf, et le transformant T30 pour la construction pBIOC21-PSSp-Lf.
L'analyse des transformants par la technique ELISA a en outre permis de mettre en évidence un taux d'expression de la rhLf dans le transformant T30 obtenu avec la construction pBIOC-PSSp-Lf trois fois supérieur à celui du transformant T19 transformé par pBIOC-PSLf-Lf. Ce résultat indique un niveau d'expression de la rhLf plus élevé lorsque le peptide signal naturel de la protéine est remplacé par celui de la sporamine.

### Technique de Western Blot.

Afin de vérifier la masse moléculaire apparente de la rhLf des transformants sélectionnés, des tests de Western Blot (Fig. 2 et 3), et d'immunoprécipitation (Fig. 4) ont été effectués à partir des extraits de protéines solubles.
Après électrophorèse en gel de polyacrylamide SDS-PAGE 7,5 % (20 µg de protéines solubles par puits), les protéines sont transférées sur membrane de nitrocellulose. Celle-ci est alors incubée dans du *PBS-tween 2 % puis lavée 3 fois dans du PBS-tween 0,05 %. Des anticorps polyclonaux de lapin ont été produits par purification d'un anti-sérum de lapin par chromatographie sur DEAE Trisacryl. Après dilution au 500^{e} dans du PBS (soit 7 µg/ml), ils ont été mis en contact avec la membrane durant 3h à 20 °C avant que celle-ci ne soit lavée dans, du PBS-tween 0,05 %. Le conjugué, un anticorps de chèvre anti-IgG de lapin (Diagnostic-Pasteur) marqué à la péroxydase et dilué au 2500^{e} dans du PBS a ensuite été incubé 1h à 20°C. La membrane a été à nouveau lavée trois fois avec du PBS. Une dernière incubation de la membrane dans 100 ml de PBS en présence de 40 mg de DAB (3-3'-diaminobenzidine tetrahydrochloride) et de 200 µl d'H₂O₂ a permis la révélation finale.
*PBS: 150 mM NaCl, 50mM Na2HPO4, NaH2PO4, pH7,5.

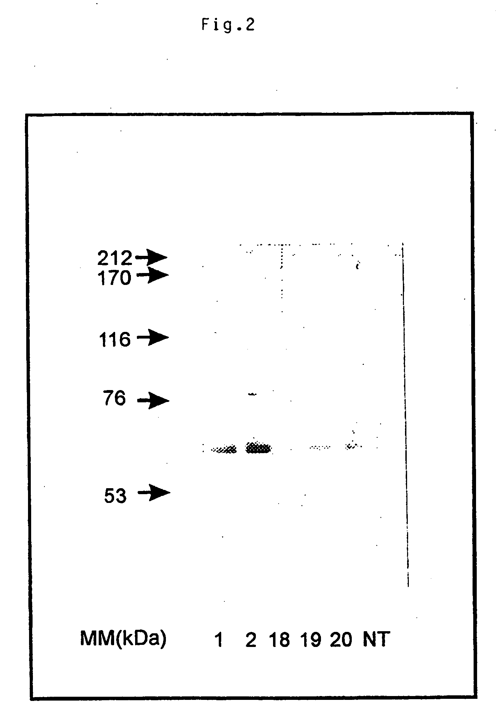

Comme le montre la Fig. 2, l'analyse des plantes issues de la première transformation montre la présence d'une bande de masse moléculaire apparente de 80 kDa reconnue par les anticorps anti-Lf. Aucun signal n'est détecté chez le témoin non transformé à savoir *Nicotiana tabacum* var. Xanthi. La présence de rhLf1 a été mise en évidence dans le transformant T19, ce qui confirme les résultats obtenus par l'analyse ELISA.

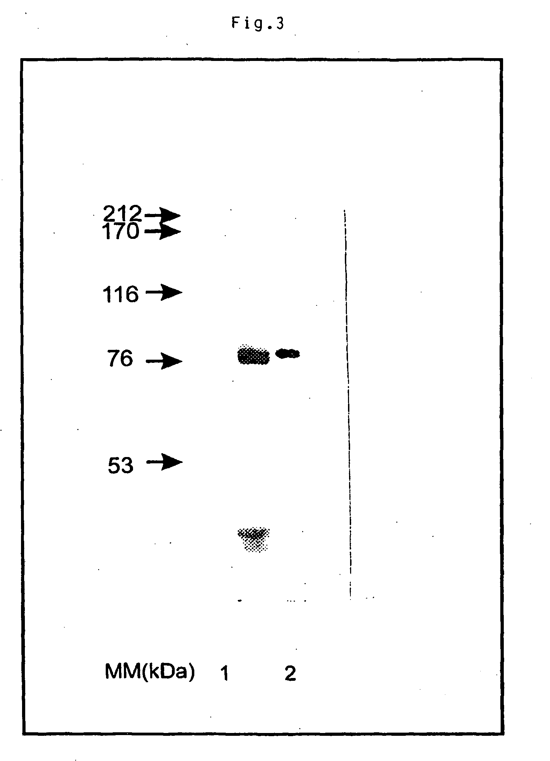

La Fig. 3 représente un Western-blot des protéines solubles issues du transformant T30. Elle démontre l'existence de rhLf de masse moléculaire apparente de 80 kDa qui co-migre avec la Lf isolée à partir du lait humain. Il est à noter que la rhLf se présente sous la forme d'une bande doublée. Ce doublet ne s'explique pas par une modification au niveau de la séquence N-terminale (cf. exemple 16).

### Immunoprécipitation

6 mg de Protéine-A Sepharose (Pharmacia) ont été incubés 1 h à température ambiante en présence de 5 mg d'anticorps polyclonaux de lapin anti-Lf humaine. Ces anticorps ont été produits comme décrit dans l'exemple. 16. Les billes de Protéine-A Sepharose ont été récupérées par centrifugation, lavées trois fois par du TBS (Tris- HCl 20 mM, NaCl 150 mM, pH8,2) et mises en agitation dans 20 ml d'extrait de protéines solubles, 2 h à température ambiante. Après trois lavages au TBS, le complexe protéique a été dissocié dans du tampon de reprise (Tris 62,5 mM, SDS 2%, saccharose 10%, β-mercaptoéthanol 5%, bleu de bromophénol 5%) séparé par gel de polyacrylamide SDS-PAGE 7,5% et enfin coloré au bleu de Coomassie.

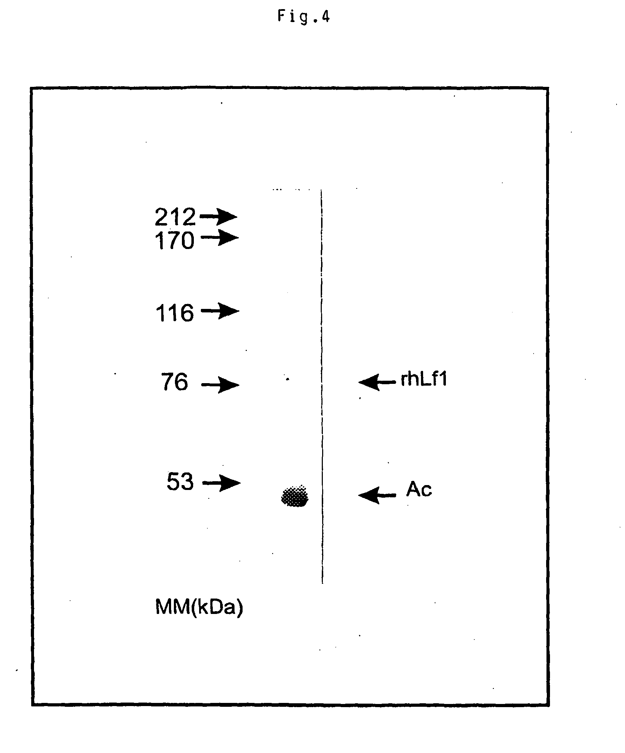

La Fig. 4 montre qu'après immunoprécipitation des extraits de protéines solubles du transformant T19, la rhLf est visualisée sous forme d'un doublet de masse moléculaire apparente de 80 kDa. Concernant le transformant T30, le résultat est identique.

### EXEMPLE 16-b : Détection des lactoferrines humaines recombinantes (rhLf) dans les graines de maïs.

### A. Extraction des protéines à partir des graines de maïs.

Les graines sont broyées dans de l'azote liquide. Puis, une macération est réalisée en ajoutant 5 ml de tampon (Tris-HCl 100mM pH8, EDTA 1mM, DTT 1mM, NaCl 250mM, Triton X100 0,2%) à 500 mg de broyat pendant une nuit à 4°C. L'homogénat est ensuite centrifugé à 10000g à 4°C pendant 10 min. Un dosage de protéines est effectué selon la méthode de Bradford.

### B. Immunodétection de la lactoferrine.

Les protéines extraites sont dénaturées par chauffage à 95°C pendant 5 min en présence du tampon Tris-HCl 50 mM pH6,8, SDS 4%, saccharose 20%, β-mercaptoéthanol 1% et bleu de bromophénol 0,01%. Les protéines sont ensuite séparées par électrophorèse sur gel de polyacrylamide en conditions dénaturantes à 10%. Après migration, les protéines sont transférées sur membrane de nitrocellulose. La lactoferrine est révélée à l'aide d'un anticorps anti-lactoferrine humain produit chez le lapin, puis d'un anti-IgG de lapin couplé à la phosphatase alcaline.

### C. Crible des graines de maïs transformées avec la construction pHMWG-IA-PSSp-Lf.

Sur les 10 transformants testés, 5 présentent un signal positif par immunodétection.

### EXEMPLE 17: Exemple de purification de la lactoferrine humaine recombinante (rhLf).

Une chromatographie d'affinité sur gel de Sepharose 4B activé au BrCN (Pharmacia Biotech) sur lequel a été branché un anticorps polyclonal anti-lactoferrine humaine a permis la purification de rhLf1 issue de pBIOC21-PSLf-Lf et de rhLf2 issue de pBIOC21-PSSp-Lf:

1 ml de gel fixant environ 3 mg d'anticorps, a été incubé pendant une nuit à 4°C en "batch" dans 100 ml d'extrait protéique dont le pH est amené à 8,2 par du Tris 1 M.

Puis, le gel a été lavé abondamment par du TBS pH 8,2 (Tris-HCl 20 mM, NaCl 150 nM, pH8,2) suivi de TBS en 1 M NaCl, pH 8,2. L'élution a été réalisée par un tampon glycine-HCl0,2 M, pH 2,4, en trois élutions successives qui sont analysées sur gel de polyacrylamide 7,5 %.

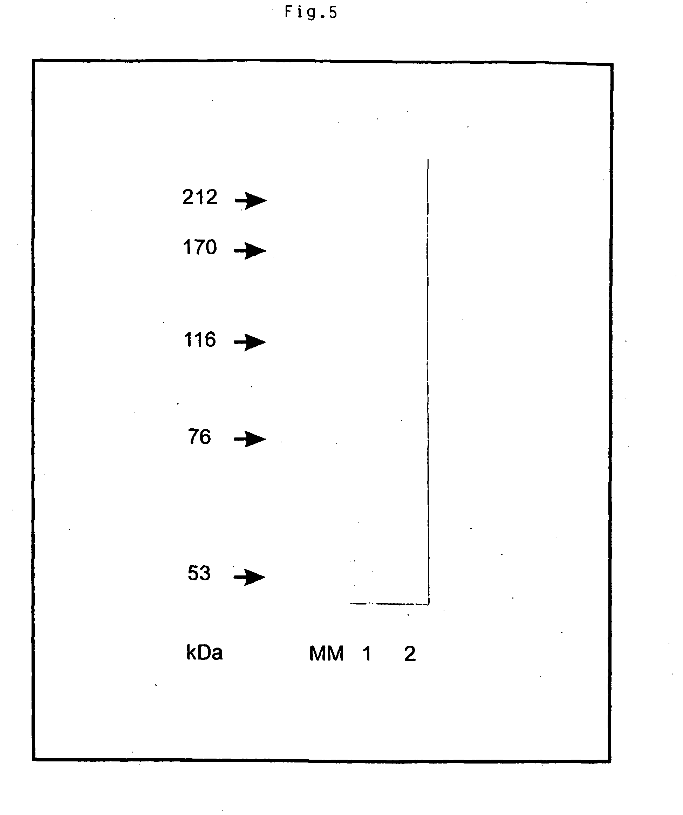

Cette technique a été utilisée pour la préparation de rhLf1 en vue de l'analyse de la séquence N-terminale. L'analyse en gel de polyacrylamide SDS-PAGE 7,5% des rhLf1 et rhLf2 purifiées par cette technique ne montre pas de différence de masse moléculaire entre les deux rhLf.(Fig. 5)

En ce qui concerne la protéine chimérique rhLf2 issue de pBIOC21-PSSp-Lf, une purification à plus grande échelle a été entreprise en vue d'analyses approfondies de cette protéine. En effet, par chromatographie d'échange d'ions sur colonne SP Sepharose Fast Flow (Pharmacia Biotech) équilibrée dans un tampon acétate de Na 0,2 M pH 7, sur un appareil de type Biopilot (Pharmacia biotech), la rhLf2 a été éluée par un gradient de NaCI de 0 à 1 M. La rhLf2 a été retrouvée majoritairement dans une fraction éluée à 0,7 M NaCl comme le prouvent les tests ELISA réalisés sur l'ensemble des fractions collectées.

Les fractions positives ont été concentrées par ultrafiltration sur Centriprep 30 (Amicon, Beverly, USA), dialysées contre du PBS (NaCl 50 mM, Na2HPO4-NaH2PO4 50 mM, pH7,5) et congelées à - 20 °C. La pureté de la rhLf2 a été vérifiée en gel de polyacrylamide SDS - PAGE 7,5 %

### EXEMPLE 18 : Variante d'extraction et de purification de la lactoferrine humaine recombinante.

Les feuilles de tabac fraîches ou congelées ont été broyées dans l'azote liquide jusqu'à l'obtention d'une poudre fine. Les protéines solubles ont ensuite été extraites à partir de la matière végétale par un tampon d'extraction (Tris/HCl 50mM,, NaCl 100mM; EDTA 1 mM, dithiothréithiol 1mm, Triton X10 0,2% (p/v), phénylméthylsulphonyl fluoride 1mm, pH 7,0) contenant 0,2 g de résine Sepabeads Diaion SP 825 (Resindion) ou Diaipn HP 20 (Supelco) par ml à raison de 4ml de tampon par gramme de matériel végétal. Le broyat a été incubé à 4°C pendant 12 heures sous agitation magnétique. Cette étape a pour but l'extraction des protéines solubles et l'adsorption d'une partie des pigments végétaux sur la résine hydrophobe. L'extrait a été centrifugé pendant 45 minutes à 15000 g puis le surnageant a été filtré sur membrane Millipore 0,45 micron.
L'extrait végétal a été chromatographié sur une colonne mono-S HR10-10 (Pharmacia Biotech) équilibrée dans une solution d'acétate de sodium 0,2M, pH 7,8 sur un appareil de type Biopilot (Pharmacia Biotech). Après passage de l'extrait sur la colonne, un gradient en NaCl de 0 à 1M dans l'acétate de sodium 0,2M a été établi sur la colonne pendant 60 minutes et la rhLf a été retrouvée dans une fraction éluant à 0,8 M NaCl. Cette fraction a été congelée à -20°C.

### EXEMPLE 19: Analyse de la séquence N-terminale.

Une analyse de la séquence N-terminale par le procédé de dégradation d'Edman a montré que pour chacune des deux constructions, c'est-à-dire pour les protéines rhLf1 et rhLf2, la séquence des 7 premiers résidus est "GRRRRSV", ce qui correspond à la séquence exacte de l'extrémité N-terminale de la lactoferrine humaine mature de référence. Ces résultats montrent que la fusion du peptide signal de sécrétion de la sporamine de patate douce ou de celui de la lactoferrine humaine à la séquence codant la lactoferrine humaine mature permet un clivage correct de ce peptide signal.

### EXEMPLE 20: Analyse en spectrométrie de masse MALDI / TOF.

Les analyses ont été réalisées sur un spectromètre de masse MALDI Vision 2000 à désorption laser (Finnigan MAT, Brenen, Allemagne). 3 µl de solution contenant 100 pmoles de lactoferrine ont été ajoutés à 17 µl de solution matrice contenant 10 mg/ml d'acide 2,5 dihydroxybenzoique dans un mélange eau/acétonitrile (30/70). 1µl de ce produit a été envoyé sur la cible, simultanément à un calibre de masse (Sérotransferrine humaine, Sigma, de masse moléculaire 79590Da dans de l'acide sinapinique). La masse moléculaire de rhLf2 est de 81250 Da, ce qui correspond à la masse calculée de la chaîne polypeptidique de la hLf (76320 Da) sur laquelle est branchée 2 glycannes de 5180 Da de masse moléculaire totale. A partir de ces valeurs, le taux de glycannes représente 6,35 % de la masse totale de la glycoprotéine.

### EXEMPLE 21: Composition des glycannes de rhLf2.

L'analyse des monosaccharides de la glycoprotéirie a été réalisée par chromatographie en phase gazeuse sur colonne capillaire OV 101 et par chromatographie sur Girdel 300. Le flux d'hélium est de 10 ml/min et la pression de 0.5 Bars. La température est programmée de 120°C à 240°C à 2°C/min. Les dérivés triméthylsylylés sont préparés par méthanolyse (Zanetta et aL, 1972), par N réacétylation et triméthylsilylation (Kamerling et al, 1975). L'analyse montre un taux de 6,5 % d'oses totaux dans la glycoprotéine. La composition molaire est décrite ci-dessous

| | Fucose | Galactose | Mannose | N-acétyl glucosami ne | Xylose | Acide N-acétyl neuraminique |
|---|---|---|---|---|---|---|
| rhLf2 | 1,5 | 0,7 | 3,0 | 3,3 | 0,7 | 0,0 |
| Lfh | 1,3 | 2,1 | 3,0 | 4,0 | 0,0 | 1,8 |

Les résultats obtenus suggèrent une structure de type N-acétyl-lactosaminique des glycannes de rhLf2 avec une certaine hétérogénéité de la composition molaire en monosaccharides. Il est à noter principalement la présence de xylose, la faible présence de galactose, et l'absence d'acide N-acétyl neuraminique .

### EXEMPLE 22: Expériences de fixations aux cellules Jurkat.

De nombreux travaux réalisés sur les lymphocytes ont permis de mettre en évidence la présence de récepteurs spécifiques de la lactoferrine à leur surface. L'apparition à la surface des lymphocytes a été étudiée par le biais. d'un agent mitogène, la phytohémagglutinine (PHA, Mazurier et al., 1989). Ces travaux ont montré que les lymphocytes quiescents ne possèdent pas de récepteur pour la lactoferrine et que la phytohémaggutinine induit l'apparition de récepteurs de haute affinité à la surface des lymphocytes circulants, récepteurs qui sont synthétisés au cours des deux premiers jours d'activation. Les constantes de fixation et le nombre de sites de fixation sont respectivement de 80 nM et 200000 nM lors des conditions d'activation à la PHA. Ce même récepteur a été étudié récemment pour la lignée lymphoblastique Jurkat qui permet d'obtenir des résultats stables et reproductibles (Bi et al., 1994). Dans l'étude de fixation de la rhLf2, cette lignée cellulaire Jurkat a été utilisée comme test d'activité biologique de la protéine recombinante.

Les cellules lymphoblastiques T de la lignée Jurkat sont cultivées en milieu RPMI 1640 (GIBCO, Cergy Pontoise, France) pH 7,4 contenant 25 mM d'Hepes, 2 mM de L glutamine, de gentamycine (5 mg / ml) en présence de 10 % de sérum de veau foetal préalablement inactivé par la chaleur, dans une étuve à 5 % de CO₂, à 37 °C.

Au stade de subconfluence, les cellules sont diluées à une densité de 4.10⁵/ml pour les expériences de fixation.

Une quantité de 100 µg de rhLf2 a été marquée par 0,2 m Ci d'¹²⁵I, en utilisant les iodobeads (Pierce, Rockford, USA) selon les recommandations du fournisseur. L'iode libre est éliminée par gel filtration sur colonne Sephadex G-25 équilibrée dans du PBS (5OmM NaCl, 50 mM Na2HP04-NaH2P04, pH 7,5). Les expériences de fixation ont été effectuées dans du RPMI contenant 0,4 % (w/v) de transferrine humaine afin d'éviter les fixations non-spécifiques de la rhLf2 sur les cellules ou sur le plastique Des aliquotes de 100 ml contenant 5.10⁵ cellules ont été réparties dans des tubes en propylène de 1,5 ml en présence de rhLf2 marquée à des concentrations croissantes de O à 100 mM.

La fixation non spécifique a été évaluée en présence de 100 excès molaire de Lf non marquée. Les cellules ont été incubées avec les protéines pendant 1 h à 4°C en présence de 0,01 % (w/v) d'azide de sodium. Enfin, les cellules ont été lavées 3 fois par 1 ml de RPMI et le culot a été finalement repris dans 0,5 ml de PBS. La radioactivité a alors été mesurée par un compteur de radiations gamma Compugamma, LKB - Wallac (Turku, Finland).

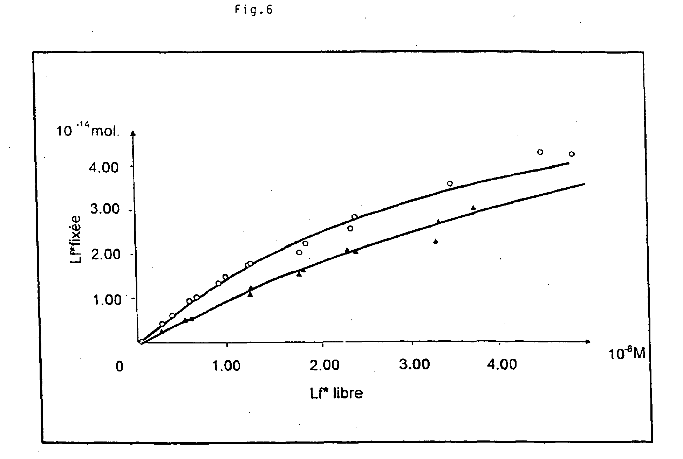

La fixation de rhLf2 marquée à l'¹²⁵I sur les cellules lymphoblastiques T Jurkat a été analysée par la méthode de Scatchard (1949). Comme le montre la Fig. 6, les courbes de fixation de rhLf2 et de la Lfh isolée à partir du lait sont similaires. Dans la gamme de Lfs utilisées, une seule classe de récepteurs est détectée à la surface des cellules avec une constante de dissociation de 80,27±33 nM et un nombre de sites par cellules de 124400±36000. Sur les mêmes cellules, la hLf se fixe avec une constante de dissociation de 89,7±22 nM et un nombre de sites par cellules de 103300±14000.

Ainsi, le test de fixation montre que rhLf2 possède une conformation très proche de celle de la Lf du lait humain puisqu'elle est reconnue avec les mêmes paramètres sur son récepteur lymphocytaire.

### EXEMPLE 23: Fixation de rhLf2 à la surface des cellules HT29.

La fixation de la lactoferrine du lait humain a été démontrée sur une lignée entérocytaire (HT-29) dérivée d'un adénocarcinome humain colique capable de se différencier.en entérocytes (Mikogami et al., 1994, 1995). Cet auteur a montré que la fixation de la lactoferrine sur son récepteur entérocytaire n'est pas liée à son degré de saturation en fer et est spécifique c'est-à-dire qu'elle ne résulte pas d'interactions électrostatiques ou lectiniques.

Sur les cellules de type HT29, le nombre de sites est de l'ordre de 3.10⁶ par cellule et le Kd d'environ 10⁻⁶ M. Le clone HT-29-18C1 répond à ces caractéristiques et a été utilisé pour la fixation de rhLf2 afin de vérifier son activité biologique.

Les cellules HT29 du clone 18Cl ont été cultivées en DMEM (Eurobio, Les Ullis, France) contenant 2 mM de L-glutamine et de la gentamycine (5 mg/l) en présence de 10% de sérum de veau foetal préalablement inactivé par la chaleur dans une étuve à 10% de CO² à 37°C. A ce stade de subconfluence, les cellules ont été fractionnées et remises en culture en puits de 2 cm², à raison de 2.10⁴ cellules/cm². Après 21 jours de culture qui permettent la différenciation en entérocytes, ces cellules ont été utilisées pour les études de fixation.

La rhLf2 a été marquée à l'¹²⁵I comme décrit dans l'exemple 19. Les cellules de chaque puits ont été incubées 1 heure sur glace en présence de 0,01% (p/v) d'azide de sodium et de transferrine humaine qui évite les fixations non spécifiques de la lactoferrine. La radioactivité d'une fraction de 50 µl été mesurée pour le calcul de la concentration du ligand libre présenté aux cellules. Après 5 rinçages de 500 µl 1 de DPBS+ (Sigma, réf. D1283), les cellules sont décollées par 200 µl de DPBS+/EDTA (5 g/l) et la radioactivité de la rhLf2 fixée est mesurée par compteur de radiations λ Compugamma, LKB-Wallac.

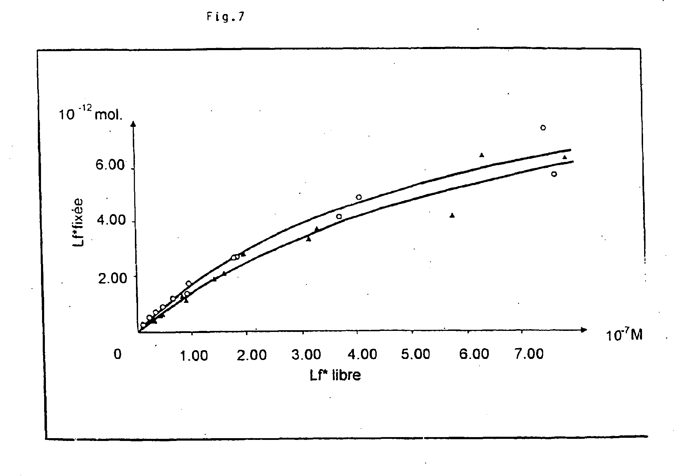

La fixation de rhLf2 marquée à l'¹²⁵I sur les cellules entérocytaires HT-29-18C1 est analysée par la méthode de Scatchard. Comme le montre la Fig. 7, les courbes de fixation des rhLf2 et Lf isolée à partir du lait sont similaires. dans la gamme des lactoferrines utilisées, une seule classe de récepteurs est détectée à la surface des cellules avec une constante de dissociation de 0,8 ±0,19 µM et un nombre de sites par cellules de 1,8x10⁶ ± 0,28x10⁶. Sur les mêmes cellules, la lactoferrine humaine se fixe avec une constante de dissociation de 1 ± 0,2 µM et un nombre de sites par cellules de 4X10⁶ ± 0,5x10⁶.

Cette fixation sur les cellules entérocytaires démontre, comme dans l'exemple 19, que rhLf2 est reconnue par le récepteur spécifique de la lactofenine de façon identique à la protéine native, ce qui suggère une bonne conformation et une activité biologique comparable à celle de la lactoferrine isolée à partir du lait.

### EXEMPLE 24: Production de lactoferrine bovine dans les plantes et leur purification.

L'utilisation des méthodes telles que décrites ci-dessus peuvent également conduire à la production de lactoferrine bovine dans les plantes.

### REFERENCES

Alexander B.F. et al, Anim.Genetics, 23, 251-256 (1992).
An et al., Plant Physiol, 81, 301-305 (1986).
An G., Plant Physiol., 81, 86-91 (1986).
Armstrong, Malze Handbook; M. Freeling, V. Walbot Eds.; pp. 665-671 (1994).
Bi B.Y. et al., Eur. J. Cell Biol., 65,164-171 (1994).
Anderson O.D. et al., Nucleic Acids Research., 17, 431-462 (1989).
Damiens et al., Biochem and Biophys. Acta 14293 (1998)
Depicker et al., J. Mol. Appl. Genet., 1, 561-573 (1982).
Depigny-This et al., Plant. Mol. Biol., 20, 467-479 (1992).
Elass-Rochard E., Roseanu A., Legrand D., Trif M., Salmon V., Motas C., Montreuil J., Spik G., 1995, 312, 839-845.
Elass-Rochard E., Legrand D., Salmon V., Roseanu A.,Trif M., Tobias P., Mazurier J., Spik G., Infection and Immunity, Feb., 486-491 (1998).
Franck et al. Cell, 21, 285-294 (1980).
Fillatti J.J. et al. Biotechnologie, 5, 726-730 (1987).
Finer J., Plant Cell Report, 11, 323-328 (1992).
Gamborg O.L. et al. Exp. Cell. Res., 50, 151-158 (1968).
Gaubier et al., Mol. Gen. Genet., 238, 409-418 (1993).
Hanahan D., J. Mol. Biol., 166, 557- 580 (1983).
Holsters et al.. Mol. Gen. Genet., 163, 181-187 (1978).
Horsch R.B. et al., Science, 227, 1229-1231 (1985).
Kay et al., Science, 236, 1299-1302 (1987).
Kamerling et al., Biochem. J. 151, 491-495 (1975).
Klein, Nature, 327, 70-73 (1987).
Laemmli U.K., Nature, 227, 680-685 (1970).
Legrand, D. et al., Biochem. J. 236, 839-844 (1986).
Le Provost F. et al, Biochem. Biophys.Res.Commun., 203, 1324-1332 (1994).
McElroy et al., Mol. Gen. Genet., 231, 150-160 (1991).
Matsuoka K. et Nakamura K., Proc. Natl. Acad. Sci. USA, 88, 834-838 (1991).
Mazurier J. et al., Eur. J. Biochem. 199, 481-487 (1989).
Metz-Boutigue M.H. et al., Eur. J. Biochem. 145, 659-676 (1984).
Mikogami T.et al., J. Am. Physiol. 267, G308-G315 (1994).
Mikogami T. et al., Biochem. J. 308, 391-397 (1995).
Mitra , Plant. Physiol. 106, 977-981 (1994)
Murakami et al., Plant Mol. Biol., 7, 343-355 (1986).
Murashige T. et Skoog F., Physiol. Plantarum, 15, 473-497 (1962).
Ni et al., Plant J., 7, 661-676 (1995).
Pierce et al, Eur.J.Biochem, 1991, 196, 177-184
Powell, M.J. et Ogden, J.E., Nucl. Acids Res. 18, 4013 (1990).
Reina et al., Nucleic Acid Research, 18, 6426 (1990).
Renart J. et Sandoval I.V., Meth. Enzymol., 104, 455-460 (1984).
Rey, M.W. et al., Nucl. Acids Res. 18, 5288 (1990).
Salmon V. et al., Prot. Express. and Purif., 13, 1998, art no.PT980886.
Scatchard, G., Ann. N.Y. Acad. Sci. 51, 660-672 (1949).
Shirsat N.V. et al, Gene, 110, 229-234 (1992).
Spik G. et al., Immunology 35, 663-671 (1978).
Spik G. et al., Eur. J. Biochem. 121, 413-419 (1982).
Vain et al., Plant Cell Tissue and Organ Culture, 18, 143-151 (1989)
Van Berkel, P.H.C. et al., Biochem. J. 319, 117-122 (1996).
Zimecki M. et al.. Archiv. Immunol. et Therap. Exper., 1996, 44, 51-56.
Zimecki M. et al., Immunol. Lett. 30, 119-124 (1991).

### DESCRIPTION DES FIGURES:

Fig. 1: Taux d'expression de rhLf1 en fonction de la guantité de protéines solubles totale (donnés en %). Colonnes 1 à 19 transformants primaires. Colonne NT : tabac non transformé.
Fig. 2: Détection de rhLf1 par techniques de Western Blot d'un extrait de protéines solubles de transformants primaires. Pistes 1, 2, 18, 19, 20: transformants primaires. Piste NT: tabac non transformé. La position des témoins de masses moléculaires est indiquée sur la gauche.
Fig. 3: Détection de rhLf2 par technique de Western Blot d'un extrait concentré de protéines solubles du transformant T30. Piste (1): analyse du transformant T30. (2) Lf humaine isolée du lait. La position des témoions de masses moléculaires est indiquée sur la gauche.
Fig. 4: Détection de rhLf1 par immunoprécipitation à partir d'un extrait de protéines solubles du transformant T19. L'analyse en SDS-PAGE met en évidence la rhLf1 et les anticorps antiLf (Ac). La position des témoins de masses moléculaires est indiquée sur la gauche.
Fig. 5: analyse de rhLf1 et rhLf2 après purification par chromatographie d'affinité.
   Piste (1): rhLf1, piste (2): rhLf2. La position des témoins de masses moléculaires est indiquée sur la gauche.
Fig. 6: Fixations spécifiques de la rhLf2 (▲) et de la hLf du lait (○) sur les cellules Iymphoblastiques Jurkat. Les Lf sont marquées à l'¹²⁵I. La fixation non spécifique est mesurée en présence de 100 excès molaire de Lf native non marquée et représente environ 25% de la fixation totale. Elle est ôtée systématiquement de la fixation totale. Ces valeurs sont déterrninées par deux expérimentations distinctes, chaque fois réalisées en double.
Fig. 7: Fixations spécifiques de la rhLf2 (▲) et de la fLf du lait (○) sur les cellules entérocytaires HT29. Les Lf sont marquées à l'¹²⁵I. La fixation non spécifique est mesurée en présence de 100 excès molaire de Lf native non marquée et représente environ 25% de la fixation totale. Elle est ôtée systématiquement de la fixation totale. Ces valeurs sont déterminées par deux expérimentations distinctes, chaque fois réalisées en double.

## Revendications

1. Utilisation d'une séquence nuclléotidique recombinante contenant
- un ADNc codant pour une lactoferrine mature.
- en amont dudit ADNc, un promoteur et en aval dudit ADNc, un terminateur de transcription reconnus par la machinerie transcriptionnelle des cellules végétales: et
- une séquence codant pour un peptide signal responsable de l'entrée des polypeptides recombinants dans le système de sécrétion des cellules végétales
pour la transformation de cellules de blé.

2. Utilisation d'une séquence nucléotidique recombinante selon la revendication 1, **caractérisée en ce que** la séquence codant pour le peptide signal est choisie dans le groupe consistant en la séquence codant le peptide signal de la lactoferrine, la séquence codant pour le peptide signal de la sporamine A de la patate douce, et la séquence codant pour le PRS.

3. Utilisation d'une séquence nucléotidique recombinante selon la revendication 1 ou 2, **caractérisée en ce que** le terminateur de transcription est choisi dans le groupe consistant en le terminateur polyA35S du virus de la mosaïque de chou-fleur (CaMV), et le terminateur polyA NOS d'*Agrobacterium tumefaciens.*

4. Utilisation d'une séquence nucléotidique recombinante selon l'une quelconque des revendications 1 à 3 **caractérisée en ce que** le promoteur est choisi dans le groupe consistant en le promoteur 35S. le promoteur constitutif double 35S (pd35S), du CaMV. le promoteur pCRU du gène de la cruciférine de radis, le promoteur pQEA1 ou pGEA6 d'*Arabidopsis thaliana,* le promoteur chimérique pSP *d'Agrobacterium tumefaciens,* le promoteur pAR-IAR du riz, le promoteur HMWG de blé, et le promoteur p-gamma-zéine de mais.

5. Utilisation d'une séquence nucléotidique recombinante selon l'une quelconque des revendications 1 à 4, **caractérisée en ce que** ledit ADNc codant pour une lactoferrine mature code pour une lactoferrine de mammifère.

6. Utilisation d'une séquence nucléotidique recombinante selon la revendication 5, ladite lactoferrine de mammifère étant la lactoferrine bovine, porcine, caprine ou humaine.

7. Séquence nucléotidique recombinante contenant :
- un ADNc codant pour une lactoferrine mature,
- en amont dudit ADNc, un promoteur et en aval dudit ADNc, un terminateur de transcription reconnus par la machinerie transcriptionnelle des cellules végétales, et
- une séquence codant pour un peptide signal responsable de l'entrée des polypeptides recombinants dans le système de sécrétion des cellules végétales.

8. Séquence nucléotidique recombinante selon la revendication 7, **caractérisée en ce que** la séquence codant pour le peptide signal est choisie dans le groupe consistant en la séquence codant le peptide signal de la lactoferrine, la séquence codant pour le peptide signal de la sporamine A de la patate douce, et la séquence codant pour te PRS.

9. Séquence nucléotidique recombinante selon la revendication 7 ou 8, **caractérisée en ce que** le terminateur de transcription est choisi dans le groupe consistant en le terminateur polyA35S du virus de la mosaïque de chou-fleur (CaMV), et le terminateur polyA NOS *d'Agrobacterium tumefaciens*.

10. Séquence nucléotidique recombinante selon l'une quelconque des revendications 7 à 9, **caractérisée en ce que** le promoteur est choisi dans le groupe consistant en le promoteur 35S, le promoteur constitutif double 35S (pd35S), du CaMV, le promoteur pCRU du gène de la cruciférine de radis, le promoteur pGEA1 ou pGEA6 *d'Arabidopsis thaliana,* le promoteur chimérique pSP *d'Agrobacterium tumefaciens,* le promoteur pAR-IAR du riz, le promoteur HMWG de blé, et le promoteur p-gamma-zéine de maïs.

11. Vecteur, notamment un plasmide, contenant une séquence nucléotidique selon rune des revendications 7 a 10 insérée en un site non essentiel pour sa réplication.

12. Hôte cellulaire, notamment bactérie telle que *Agrobacterium tumefaciens*, transformé par un vecteur selon la revendication 11.

13. Cellule de maïs, **caractérisée en ce qu'**elle contient, intégrée de façon stable dans son génome, une séquence nucléotidique recombinante contenant
- un ADNc codant pour une lactoferrine mature,
- en amont dudit ADNc, un promoteur et en aval dudit ADNc, un terminateur de transcription reconnus par la machinerie transcriptionnelle des cellules végétales, et
- une séquence codant pour un peptide signal responsable de l'entrée des polypeptides recombinants dans le système de sécrétion des cellules végétales.

14. Plante de maïs, obtenue à partir d'une cellule selon la revendication 13.

15. Extrait ou partie d'une plante selon la revendication 14, notamment feuilles et/ou fruits et/ou semences.

16. Procédé d'obtention de la lactoferrine, **caractérisé en ce qu'**il comprend la récupération de la lactoferrine recombinante produite dans les cellules, plantes ou parties de plantes transformées selon les revendications 13 à 15, notamment par extraction, suivie, le cas échéant par une purification.

17. Procédé selon la revendication 16, ladite lactoferrine étant la lactoferrine humaine.

18. Utilisation de plantes, extraits de plantes ou parties de plantes selon la revendication 14 ou 15, pour l'obtention de compositions pharmaceutiques, médicales, odontologiques, cosmétiques ou biotechnologiques.

19. Biomatériau ou composition pharmaceutique, médicale, odontologique, cosmétique ou biotechnologique, **caractérisée en ce qu'**elle comprend des plantes, extraits de plante, parties de plante selon la revendication 14 ou 15.

## Claims

1. Use of a recombinant nucleotide sequence containing
- a cDNA coding for a mature lactoferrin,
- upstream of said cDNA a promoter and downstream of said cDNA a transcription terminator recognised by the transcriptional machinery of plant cells, and
- a sequence coding for a signal peptide responsible for entry of the recombinant polypeptides in the secretion system of the plant cells
for the transformation of maize cells.

2. Use of a recombinant nucleotide sequence according to claim 1, **characterised in that** the sequence coding for the signal peptide is selected from the group consisting of the sequence encoding the signal peptide of lactoferrin, the sequence coding for the signal peptide of sweet potato sporamin A, and the sequence coding for PRS.

3. Use of a recombinant nucleotide sequence according to either claim 1 or claim 2, **characterised in that** the transcription terminator is selected from the group consisting of the terminator polyA 35S of the cauliflower mosaic virus (CaMV) and the terminator polyA NOS of Agrobacterium tumefaciens.

4. Use of a recombinant nucleotide sequence according to any one of claims 1 to 3, **characterised in that** the promoter is selected from the group consisting of the promoter 35S, the double constitutive promoter (Pd35S) of the CaMV, the promoter PCRU of the radish cruciferin gene, the promoter pGBA1 or pGEA6 of Arabidopsis thaliana, the chimeric promoter PSP of Agrobacterium tumefaciens, the rice promoter pAR-IAR, the barley HMWG promoter, and the maize p-γ-zein promoter.

5. Use of a recombinant nucleotide sequence according to any one of claims 1 to 4, **characterised in that** said cDNA coding for a mature lactoferrin codes for a mammalian lactoferrin.

6. Use of a recombinant nucleotide sequence according to claim 5, said mammalian lactoferrin being bovine, porcine, caprine or human lactoferrin.

7. Recombinant nucleotide sequence containing:
- a cDNA coding for a mature lactoferrin,
- upstream of said cDNA a promoter and downstream of said cDNA a transcription terminator recognised by the transcriptional machinery of plant cells, and
- a sequence coding for a signal peptide responsible for entry of the recombinant polypeptides in the secretion system of the plant cells.

8. Recombinant nucleotide sequence according to claim 7, **characterised in that** the sequence coding for the signal peptide is selected from the group consisting of the sequence encoding the signal peptide of lactoferrin, the sequence coding for the signal peptide of sweet potato sporamin A, and the sequence coding for PRS.

9. Recombinant nucleotide sequence according to either claim 7 or claim 8, **characterised in that** the transcription terminator is selected from the group consisting of the terminator polyA 35S of the cauliflower mosaic virus (CaMV) and the terminator polyA NOS of Agrobacterium tumefaciens.

10. Recombinant nucleotide sequence according to any one of claims 7 to 9, **characterised in that** the promoter is selected from the group consisting of the promoter 35S, the double constitutive promoter (Pd35S) of the CaMV, the promoter PCRU of the radish cruciferin gene, the promoter pGEA1 or pGEA6 of Arabidopsis thaliana, the chimeric promoter PSP of Agrobacterium tumefaciens, the rice promoter pAR-IAR, the barley HMWG promoter, and the maize p-γ-zein promoter.

11. Vector, particularly a plasmid, containing a nucleotide sequence according to any one of claims 7 to 10 inserted at a site which is not essential for its replication.

12. Cellular host, particularly a bacterium such as Agrobacterium tumefaciens, transformed by a vector according to claim 11.

13. Maize cell, **characterised in that** it contains, integrated in a stable manner in the genome thereof, a recombinant nucleotide sequence containing
- a cDNA coding for a mature lactoferrin,
- upstream of said cDNA a promoter and downstream of said cDNA a transcription terminator recognised by the transcriptional machinery of plant cells, and
- a sequence coding for a signal peptide responsible for entry of the recombinant polypeptides in the secretion system of the plant cells.

14. Maize plant obtained from a cell according to claim 13.

15. Extract or part of a plant according to claim 14, particularly leaves and/or fruits and/or seeds.

16. Method for producing lactoferrin, **characterised in that** it comprises recovery of recombinant lactoferrin produced in the transformed cells, plants or parts of plants according to claims 13 to 15, particularly by extraction followed, as necessary, by purification.

17. Method according to claim 16, said lactoferrin being human lactoferrin.

18. Use of plants, plant extracts or parts of plants according to either claim 14 or claim 15, for the production of pharmaceutical, medical, odontological, cosmetic or biotechnological compositions.

19. Biomaterial or a pharmaceutical, medical, odontological, cosmetic or biotechnological composition, **characterised in that** it comprises plants, plant extracts, parts of plants according to either claim 14 or claim 15.

## Patentansprüche

1. Verwendung einer rekombinanten Nukleotidsequenz, umfassend:
- eine cDNA, die codierend für ein reifes Lactoferrin ist,
- stromaufwärts von der cDNA einen Promotor und stromabwärts von der cDNA einen Transkriptionsterminator, die von der Transkriptionsmaschinerie der Pflanzenzellen erkannt werden, und
- eine Sequenz, die codierend für ein Signalpeptid ist, das für den Eingang der rekombinanten Polypeptide in das Sekretionssystems der Pflanzenzellen verantwortlich ist
zur Transformation von Maiszellen.

2. Verwendung einer rekombinanten Nukleotidsequenz nach Anspruch 1, **dadurch gekennzeichnet, dass** die Sequenz, die codierend für das Signalpeptid ist, in der Gruppe gewählt wird, die gebildet wird aus der Sequenz, die das Signalpeptid von Lactoferrin codiert, aus der Sequenz, die codierend für das Signalpeptid von Sporamin A der Süßkartoffel ist, und aus der Sequenz, die codierend für PRS ist.

3. Verwendung einer rekombinanten Nukleotidsequenz nach Anspruch 1 oder 2, **dadurch gekennzeichnet, dass** der Transkriptionsterminator in der Gruppe gewählt wird, die gebildet wird aus dem Terminator polyA35S des Blumenkohl-Mosaikvirus (CaMV) und aus dem Terminator polyA NOS von *Agrobacterium tumefaciens.*

4. Verwendung einer rekombinanten Nukleotidsequenz nach einem der Ansprüche 1 bis 3, **dadurch gekennzeichnet, dass** der Promotor in der Gruppe gewählt wird, die gebildet wird aus dem Promotor 35S, aus dem konstitutiven zweifachen Promotor 35S (pd35S) von CaMV, aus dem Promotor pCRU des Gens von Rettich-Cruciferin, aus dem Promotor pGEA1 oder pGEA6 von *Arabidopsis thaliana,* aus dem chimerischen Promotor pSP von *Agrobacterium tumefaciens,* aus dem Promotor pAR-IAR von Reis, aus dem Promotor HMWG von Weizen und aus dem Promotor p-Gamma-Zein von Mais.

5. Verwendung einer rekombinanten Nukleotidsequenz nach einem der Ansprüche 1 bis 4, **dadurch gekennzeichnet, dass** die cDNA, die codierend für ein reifes Lactoferrin ist, ein Lactoferrin eines Säugetieres codiert.

6. Verwendung einer rekombinanten Nukleotidsequenz nach Anspruch 5, wobei das Säugetier-Lactoferrin Lactoferrin des Rinds, des Schweins, der Ziege oder des Menschen ist.

7. Rekombinante Nukleotidsequenz, umfassend:
- eine cDNA, die codierend für ein reifes Lactoferrin ist,
- stromaufwärts von der cDNA einen Promotor und stromabwärts von der cDNA einen Transkriptionsterminator, die von der Transkriptionsmaschinerie der Pflanzenzellen erkannt werden, und
- eine Sequenz, die codierend für ein Signalpeptid ist, das für den Eingang der rekombinanten Polypeptide in das Sekretionssystems der Pflanzenzellen verantwortlich ist.

8. Rekombinante Nukleotidsequenz nach Anspruch 7, **dadurch gekennzeichnet, dass** die Sequenz, die codierend für das Signalpeptid ist, in der Gruppe gewählt wird, die gebildet wird aus der Sequenz, die das Signalpeptid von Lactoferrin codiert, aus der Sequenz, die codierend für das Signalpeptid von Sporamin A der Süßkartoffel ist, und aus der Sequenz, die codierend für PRS ist.

9. Rekombinante Nukleotidsequenz nach Anspruch 7 oder 8, **dadurch gekennzeichnet, dass** der Transkriptionsterminator in der Gruppe gewählt wird, die gebildet wird aus dem Terminator polyA35S des Blumenkohl-Mosaikvirus (CaMV) und aus dem Terminator polyA NOS von *Agrobacterium tumefaciens.*

10. Rekombinante Nukleotidsequenz nach einem der Ansprüche 7 bis 9, **dadurch gekennzeichnet, dass** der Promotor in der Gruppe gewählt wird, die gebildet wird aus dem Promotor 35S, aus dem konstitutiven zweifachen Promotor 35S (pd35S) von CaMV, aus dem Promotor pCRU des Gens von Rettich-Cruciferin, aus dem Promotor pGEA1 oder pGEA6 von *Arabidopsis thaliana,* aus dem chimerischen Promotor pSP von *Agrobacterium tumefaciens,* aus dem Promotor pAR-IAR von Reis, aus dem Promotor HMWG von Weizen und aus dem Promotor p-Gamma-Zein von Mais.

11. Vektor, insbesondere ein Plasmid, umfassend eine Nukleotidsequenz nach einem der Ansprüche 7 bis 10, die an einer Stelle eingefügt ist, die für ihre Replikation nicht wesentlich ist.

12. Zellulärer Wirt, insbesondere ein Bakterium wie *Agrobacterium tumefaciens,* das durch einen Vektor nach Anspruch 11 transformiert wurde.

13. Maiszelle, **dadurch gekennzeichnet, dass** sie auf stabile Weise in ihr Genom integriert eine rekombinante Nukleotidsequenz enthält, die umfasst:
- eine cDNA, die codierend für ein reifes Lactoferrin ist,
- stromaufwärts von der cDNA einen Promotor und stromabwärts von der cDNA einen Transkriptionsterminator, die von der Transkriptionsmaschinerie der Pflanzenzellen erkannt werden, und
- eine Sequenz, die codierend für ein Signalpeptid ist, das für den Eingang der rekombinanten Polypeptide in das Sekretionssystems der Pflanzenzellen verantwortlich ist.

14. Maispflanze, die ausgehend von einer Zelle nach Anspruch 13 erzielt wird.

15. Extrakt oder Teil einer Pflanze nach Anspruch 14, insbesondere Blätter und/oder Früchte und/oder Samen.

16. Verfahren zur Herstellung von Lactoferrin, **dadurch gekennzeichnet, dass** es die Wiedergewinnung des rekombinanten Lactoferrins umfasst, das in den transformierten Zellen, Pflanzen oder Pflanzenteilen nach den Ansprüchen 13 bis 15 produziert wird, und zwar insbesondere durch Extraktion, gegebenenfalls gefolgt von einer Reinigung.

17. Verfahren nach Anspruch 16, wobei das Lactoferrin menschliches Lactoferrin ist.

18. Verwendung von Pflanzen, Pflanzenextrakten oder Pflanzenteilen nach Anspruch 14 oder 15 zur Herstellung von pharmazeutischen, medizinischen, zahnheilkundlichen, kosmetischen oder biotechnologischen Zusammensetzungen.

19. Biomaterial oder pharmazeutische, medizinische, zahnheilkundliche, kosmetische oder biotechnologische Zusammensetzung, **dadurch gekennzeichnet, dass** sie Pflanzen, Pflanzenextrakte oder Pflanzenteile nach Anspruch 14 oder 15 umfasst.
